# EUROPEAN PATENT APPLICATION

(11) **EP 4 342 526 A1**
(43) Date of publication of application: **27.03.2024**
(21) Application number: 22827265.4
(22) Date of filing: 19.05.2022
(51) Int. Cl.: A61N 5/10, A61B 34/30

(54) **AUTOMATED PARTICLE IMPLANTATION SYSTEM, PARTICLE CHAIN GENERATION MECHANISM, AND PUNCTURE DEVICE**

(30) Priority: 24.06.2021 CN 202110707539; 27.01.2022 CN 202210102528; 27.01.2022 CN 202220230592 U
(71) Applicant: Wuhan United Imaging Healthcare Surgical Technology Co., Ltd., Wuhan, Hubei 430073 (CN)
(72) Inventor: ZHAO, Zhuo, Wuhan, Hubei 430073 (CN); SUN, Hongyu, Shanghai 201807 (CN)
(74) Representative: Wang, Bo
(86) International application number: PCT/CN2022/093757
(87) International publication number: WO 2022/267767

(57) **Abstract**

An automated particle implantation system (41), comprising an active system (411) and a passive system (412), wherein the active system (411) is configured to: acquire a scanning image of a scanned object (S110); perform radioactive particle implantation treatment planning according to the scanned image, so as to obtain a radioactive particle implantation treatment planning result (S120); and according to the radioactive particle implantation treatment planning result, control the passive system (412) to execute a radioactive particle implantation operation (S130). Further disclosed in the embodiments of the description are a particle chain generation mechanism and a puncture device terminal.

## Description

### CROSS REFERENCE TO RELATED APPLICATIONS

The present disclosure claims priority to Chinese Patent Application No. 202110707539.0, entitled "surgical robotics for radioactive particle implantation and system thereof, filed on June 24, 2021, Chinese Patent Application No. 202210102528.4, entitled "surgical robotics, and particle implantation mechanism and robotic arm terminal device for surgical robotics", filed on January 27, 2022, and Chinese Patent Application No. 202220230592.6, entitled "surgical robotics and terminal mechanism for surgical robotics", filed on January 27, 2022, the entire contents of each of which are hereby incorporated by reference.

### TECHNICAL FIELD

The present disclosure relates to the technical field of medical devices, and in particular, to an automated particle implantation system, a particle chain generating mechanism, and a puncture device for radioactive particle implantation.

### BACKGROUND

Radioactive particle implantation treatment technology is a methodology that accurately implants a radioactive particle into a tumor, which operates as a micro-radioactive source that emits continuous, short-distance radiation. This methodology allows tumor tissue to suffer maximum damage while ensuring normal tissue is not damaged or only slightly damaged. Radioactive particle implantation has the advantages of requiring a short treatment cycle, having limited requirements for on-site devices, being economically priced, and causing less damage to healthy tissues adjacent to the tumor. In state-of-the-art technologies, the process of radioactive particle implantation constitutes: (1) A doctor determines a radiation dose and a trajectory for the radioactive particle in an independent treatment planning system (TPS) based on a patient's scan images and orders radioactive particles from a radioactive particle manufacturer accordingly. The ordering process usually takes 2 to 3 days. (2) After obtaining the radioactive particles, the doctor performs a manual puncture accordingly and placing the radioactive particles at the predetermined position. This kind of radioactive particle implantation process requires a long treatment cycle and has a high time-cost and low operation efficiency.

At present, no effective solution has been proposed for the problems associated with start-of-the-art technologies such as requiring a long treatment cycle, cumbersome surgical procedures, long surgical time, and low surgical efficiency during the radioactive particle implantation. Therefore, it is necessary to introduce an automated particle implantation system, a particle-chain-generating mechanism, and a puncture device.

### SUMMARY

The present disclosure discloses an automated particle implantation system, comprising an active system and a passive system, the active system may be configured to: obtain a scanning image of a scanned object; obtain a radioactive particle implantation treatment planning result by planning a radioactive particle implantation treatment according to the scanning image ; and control the passive system to execute a radioactive particle implantation operation according to the radioactive particle implantation treatment planning result.

In some embodiments, in order to obtain the scanning image of the scanned object, the active system may be further configured to control a scanning device to scan the scanned object.

In some embodiments, in order to obtain the radioactive particle implantation treatment planning result by planning the radioactive particle implantation treatment according to the scanning image, the active system may be further configured to determine particle implantation path information based on the scanning image, a target dose, and/or a motion constraint condition of a robotic arm.

In some embodiments, in order to determine the particle implantation path information based on the scanning image, the target dose, and/or the motion constraint condition of the robotic arm, the active system may be further configured to determine a region of interest in the scanning image; and determine the particle implantation path information according to the region of interest, target dose, and/or motion constraint condition of the robotic arm.

In some embodiments, in order to determine the particle implantation path information according to the region of interest, target dose, and/or motion constraint condition of the robotic arm, the active system may be further configured to obtain initial particle implantation path information according to a size, a shape and/or position of the region of interest, and/or the target dose; and determine target particle implantation path information corresponding to the scanned object by verifying the initial particle implantation path information according to the motion constraint condition of the robotic arm.

In some embodiments, in order to verify the initial particle implantation path information according to the motion constraint condition of the robotic arm and determine the target particle implantation path corresponding to the scanned object, the active system may be further configured to: in response to a determination that the verification passes, determine the initial particle implantation path information as the target particle implantation path information corresponding to the scanned object; and in response to a determination that the verification fails, correct the initial particle implantation path information according to the region of interest, target dose, and/or motion constraint condition of the robotic arm until the verification is passed, and obtain the target particle implantation path information.

In some embodiments, the particle implantation path information may include: a count of puncture needle assemblies, a needle insertion position of each of the puncture needle assemblies, a target point position of the each puncture needle assembly, a count of particles at the target point position of the each puncture needle assembly, and/or a moving path of the robotic arm.

In some embodiments, the active system may be further configured to verify one or more pieces of information of the particle implantation path information.

In some embodiments, the passive system may be configured to execute the radioactive particle implantation operation according to the radioactive particle implantation treatment planning result, the radioactive particle implantation operation comprising: moving a robotic arm to a needle insertion position; inserting a puncture needle assembly into a target point position; implanting one or more particles; withdrawing the puncture needle assembly; and implanting one or more particles using a next puncture needle assembly, until all puncture needle assemblies in the radioactive particle implantation treatment planning result have been used to finish particle implantation.

In some embodiments, the active system may be further configured to: after all puncture needle assemblies in the radioactive particle implantation treatment planning result are used to finish particle implantation, control a scanning device to scan the scanned object to test an implantation effect.

In some embodiments, wherein the passive system may include a puncture device, the puncture device may include a puncture device terminal and at least one puncture needle assembly mounted on the puncture device terminal, and a size and shape of the puncture device terminal may be related to a count of the at least one puncture needle assembly, and each of the at least one puncture needle assembly may be configured to establish a particle implantation channel.

In some embodiments, the active system may include a radiation planning module, and the radiation planning module may be configured to determine a count of particles according to a target dose; the passive system may include a particle chain generating mechanism, and the particle chain generating mechanism may be configured to form a particle chain in the particle implantation channel according to the count of particles and a space-occupying material in the radioactive particle implantation operation.

In some embodiments, in order to control the passive system to execute the radioactive particle implantation operation according to the radioactive particle implantation treatment planning result, the active system may be further configured to: control the passive system to establish a first particle implantation channel through an outer needle of the puncture device, and inject a first particle chain into the first particle implantation channel; and after the first particle implantation channel is established, control the passive system to establish a second particle implantation channel through an inner needle of the puncture device, and inject a second particle chain into the second particle implantation channel.

In some embodiments, in order to obtain the radioactive particle implantation treatment planning result by planning the radioactive particle implantation treatment based on the scanning image, the active system may be further configured to obtain a body surface position of the scanned object through a scanning device; and in response to a determination that a change amount of the body surface position within a preset time is greater than or equal to a preset change threshold, modify the radioactive particle implantation treatment planning result according to a current scanning image.

In some embodiments, the active system may include a display device, the display device may be configured to display at least one of the scanning image, the radioactive particle implantation treatment planning result, a remaining amount of the particles to be implanted, or a motion state of the passive system.

The second aspect of the embodiment of the present disclosure discloses a particle chain generating mechanism, comprising a storage unit and an implanting assembly; the storage unit may be configured to store at least two materials used to form a particle chain, and at least a portion of the implanting assembly may be inserted into the storage unit and can selectively cause the at least two materials located in the storage unit to form the particle chain in an orderly manner.

In some embodiments, the at least two materials may include a radioactive particle and a space-occupying material.

In some embodiments, the storage unit may include a first material clamp and a second material clamp, the first material clamp may be configured to store the radioactive particle, and the second material clamp may be configured to store the space-occupying material.

In some embodiments, the implanting assembly may be selectively connected to the first material clamp or the second material clamp.

In some embodiments, at least a portion of the implanting assembly may be rotatably connected to the storage unit, so as to be selectively connected to the first material clamp or the second material clamp.

In some embodiments, the implanting assembly may include an implanting trocar and a push rod, and the implanting trocar may be rotatably connected to the storage unit, an end of the push rod extends into the implanting trocar and at least two included angles between the storage unit may be formed as the implanting trocar rotates, so as to correspond to the first material clamp or the second material clamp.

The third aspect of the embodiment of the present disclosure discloses a puncture device terminal, comprising a switching frame and a switching frame driving mechanism, wherein the switching frame driving mechanism may include a transmission assembly, and the transmission assembly may be configured to drive a puncture needle assembly to move to a puncture needle interface.

In some embodiments, the puncture needle assembly may be driven by the transmission assembly to move to the puncture needle interface in a translational or rotational manner.

In some embodiments, the switching frame may be provided with an installation slot, and the installation slot may be configured to install the at least two puncture needle assemblies including the puncture needle assembly.

In some embodiments, a cross-section of the installation slot may have an annular shape, a spiral shape or a shape of character " 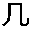 ".

In some embodiments, the puncture device terminal further includes a puncture needle driving device. The puncture needle driving device may include a particle chain generating mechanism and a trocar driving mechanism, the particle chain generating mechanism is configured to provide a particle chain for the puncture needle assembly, and the trocar driving mechanism may be configured to drive the puncture needle assembly to perform a puncture operation.

In some embodiments, the puncture needle assembly may include an outer needle and an inner needle, and the outer needle may encase the inner needle.

In some embodiments, the puncture needle assembly may further include a first driving unit and a second driving unit, the first driving unit may connect and drive the outer needle and the inner needle to perform the puncture operation, and the second driving unit may connect and drive the inner needle to release the particle chain provided by the particle chain generating mechanism.

The fourth aspect of the embodiment of the present disclosure discloses a puncture device terminal, comprising a switching frame and a switching frame driving mechanism, wherein the switching frame may be provided with an installation slot and a puncture needle interface connected to the installation slot, the installation slot may be configured to install at least one puncture needle assembly, and the switching frame driving mechanism may be configured to switch each of the at least one puncture needle assembly to the puncture needle interface.

In some embodiments, a path of the switching frame driving mechanism switching each of the at least one puncture needle assembly may include a spiral line.

In some embodiments, the switching frame may have an installation surface with a spiral cross-section, and the installation slot may be provided on the installation surface.

In some embodiments, the installation surface may include a first installation surface and a second installation surface, the first installation surface and the second installation surface may be located on inner and outer sides of the switching frame, respectively.

In some embodiments, an opening position of the installation surface may correspond to a position of the puncture needle interface, or a center position of the spiral line may correspond to the position of the puncture needle interface.

The fifth aspect of the embodiment of the present disclosure discloses a puncture device terminal, comprising: a switching frame, the switching frame being configured to connect with a robotic arm; at least two installation slots, the at least two installation slots being provided in the switching frame and being configured to accommodate at least two corresponding puncture needle assemblies; a switching frame driving mechanism, the switching frame driving mechanism being provided in the switching frame, and being configured to drive the at the least two puncture needle assemblies to switch; and a puncture needle driving device, the puncture needle driving device being configured to drive the puncture needle assembly to perform one or more puncture operations.

In some embodiments, the switching frame may rotate under the driving of the switching frame driving mechanism.

In some embodiments, the switching frame driving mechanism may include a rotating shaft, a transmission unit and a driving component, and the rotating shaft may be fixedly installed on the switching frame, the transmission unit may be connected to the rotating shaft and the driving component.

In some embodiments, the transmission unit may include at least one of synchronous belt transmission, a chain transmission, or a gear transmission.

In some embodiments, each of the at least two puncture needle assemblies may include an outer needle and an inner needle, the outer needle may be accommodated in the installation slot, the inner needle may be inserted into the outer needle and can move along an axial direction of the outer needle.

In some embodiments, the puncture needle driving device may include a first driving unit and a second driving unit arranged independently from each other, and the first driving unit may drive the outer needle and the inner needle, and the second driving unit may drive the inner needle.

In some embodiments, the first driving unit and the second driving unit may be screw drive structures.

In some embodiments, the first driving unit and the second driving unit may be electrically connected to the robotic arm, respectively.

In some embodiments, the puncture device terminal may further include an electrical interface, wherein the electrical interface may be provided on the switching frame, and one end of the electrical interface may be electrically connected to the first driving unit and the second driving unit respectively, and the other end of the electrical interface may be electrically connected to the robotic arm.

In some embodiments, the puncture needle driving device may be provided on the switching frame.

In some embodiments, the at least two installation slots may be driven by the switching frame driving mechanism.

In some embodiments, the at least two installation slots may be arranged in an annular shape, a spiral or a shape of character " 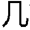 ".

### BRIEF DESCRIPTION OF THE DRAWINGS

The drawings described here are used to provide a further understanding of the present disclosure as a part of the present disclosure. The illustrative embodiments and descriptions of the present disclosure are used to explain the present disclosure and do not constitute an improper limitation of the present disclosure.
FIG. 1 is a flowchart illustrating an exemplary process of radioactive particle implantation according to some embodiments of the present disclosure;
FIG. 2 is a flowchart illustrating an exemplary process of target dose and implantation path planning according to some embodiments of the present disclosure;
FIG. 3 is a flowchart illustrating an exemplary process of radioactive particle implantation according to some embodiments of the present disclosure;
FIG. 4 is a schematic diagram illustrating an exemplary structure of an automated particle implantation robotic system according to some embodiments of the present disclosure;
FIG. 5 is a flowchart illustrating another process of radioactive particle implantation according to some embodiments of the present disclosure;
FIG. 6 is a schematic diagram illustrating an exemplary structure of an automated particle implantation system according to some embodiments of the present disclosure;
FIG. 7 is a schematic diagram illustrating an exemplary hardware structure of a radioactive particle implantation terminal according to some embodiments of the present disclosure;
FIG. 8 is a schematic diagram illustrating an exemplary structure of a particle chain generating mechanism according to some embodiments of the present disclosure;
FIG. 9A is a schematic diagram illustrating an exemplary structure of a second material clamp in a particle chain generating mechanism shown in FIG. 8;
FIG. 9B is a schematic diagram illustrating an exemplary structure of a particle chain generated by a particle chain generating mechanism shown in FIG. 8;
FIG. 10 is a schematic diagram illustrating an exemplary structure of a puncture needle driving device according to some embodiments of the present disclosure;
FIG. 11 is a schematic diagram illustrating an exemplary structure of the puncture needle driving device shown in FIG. 10 after being installed;
FIG. 12 is a schematic diagram illustrating an exemplary structure of a puncture needle assembly according to some embodiments of the present disclosure;
FIGs. 13A and 13B are schematic diagrams illustrating an exemplary structure of a puncture device terminal according to some embodiments of the present disclosure;
FIG. 14 is a schematic diagram illustrating an exemplary structure of a puncture device terminal according to some embodiments of the present disclosure;
FIG. 15 is a schematic diagram illustrating an exemplary structure of a puncture device terminal according to some embodiments of the present disclosure;
FIG. 16 is a schematic diagram illustrating an exemplary " 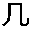 " shaped cross-section of an installation slot of a switching frame in the puncture device terminal shown in FIGs. 13A and 13B;
FIG. 17 is a schematic diagram illustrating an exemplary structure of a puncture device terminal according to some embodiments of the present disclosure.

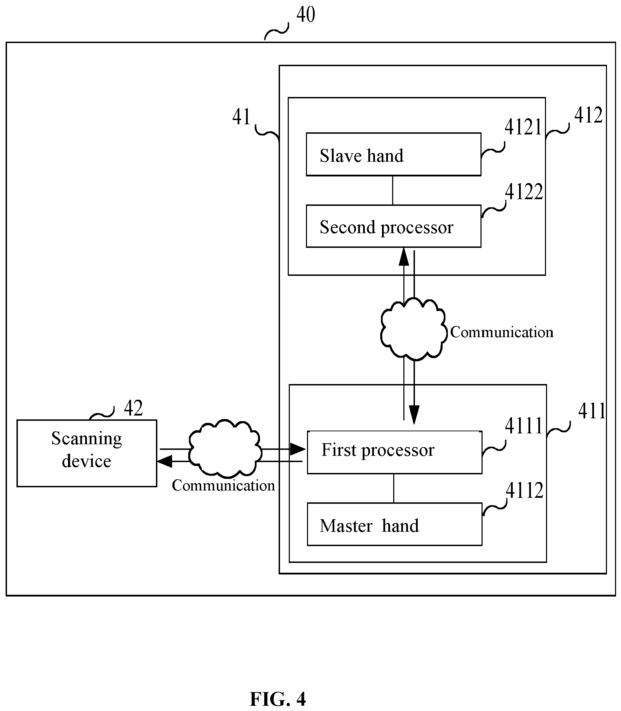

Description of reference numbers in FIG. 4: 40, automated particle implantation robotic system; 41, automated particle implantation system; 42, scanning device; 411, active system; 412, passive system; 4111, first processor; 4112, master hand; 4121, slave hand; 4122, second processor.

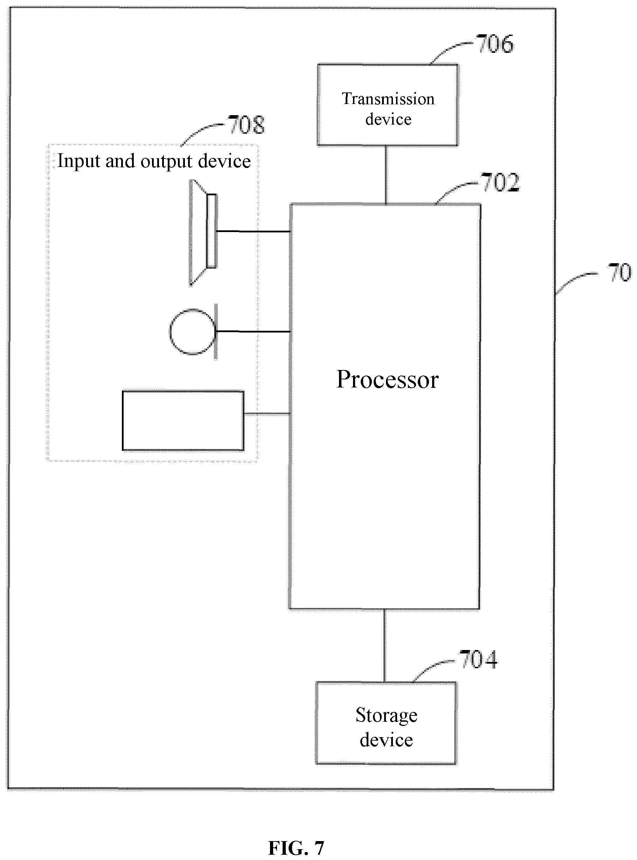

Description of reference numbers in FIG. 7: 70, terminal for radioactive particle implantation; 702, processor; 704, memory; 706, transmission device; 708, input and output device.

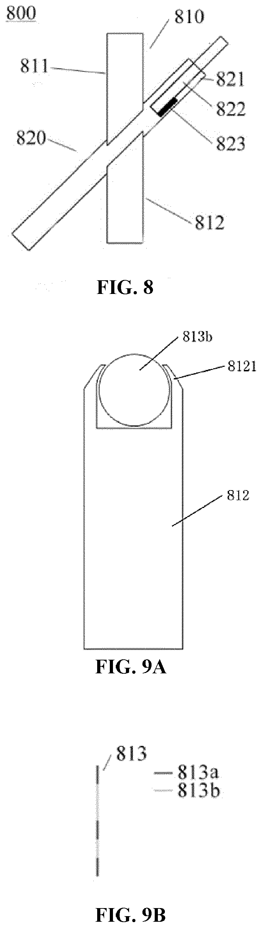

Description of reference numbers in FIG. 8: 800, particle chain generating mechanism; 810, storage unit; 811, first material clamp; 812, second material clamp; 820, implanting assembly; 821, implanting trocar; 822, push rod; 823, eccentric block.

Description of reference numbers in FIG. 9A: 813b, space-occupying material; 8121, position-limiting part.

Description of reference numbers in FIG. 9B denote: 813, particle chain; 813a, radioactive particle; 813b, space-occupying material.

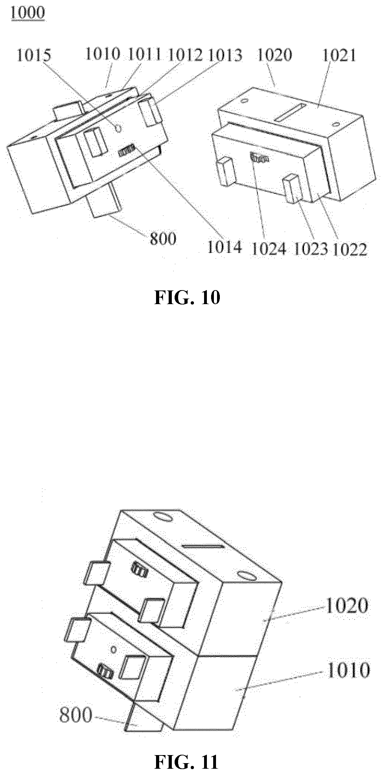

Description of reference numbers in FIG. 10 and FIG. 11: 1000, puncture needle driving device; 1010, first drive assembly; 1011, first housing; 1015, output channel; 1013, first clamping part; 1014, first gear; 1020, second driving assembly; 1021, second housing; 1023, second clamping part; 1024, second gear; 1012, first telescopic module; 1022, second telescopic module; 800, particle chain generating mechanism.

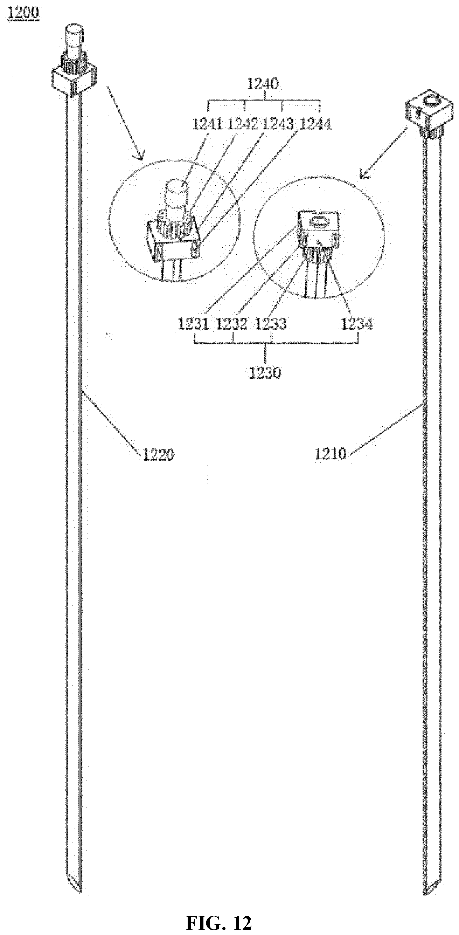

Description of reference numbers in FIG. 12 : 1200, puncture needle assembly; 1210, outer needle; 1220, inner needle; 1230, first driving unit; 1231, outer needle installation frame; 1232, first clamping slot; 1233, outer needle gear; 1234, implantation port; 1240, second driving unit; 1241, telescopic positioning column; 1242, inner needle gear; 1243, inner needle installation frame; 1244, second clamping slot.

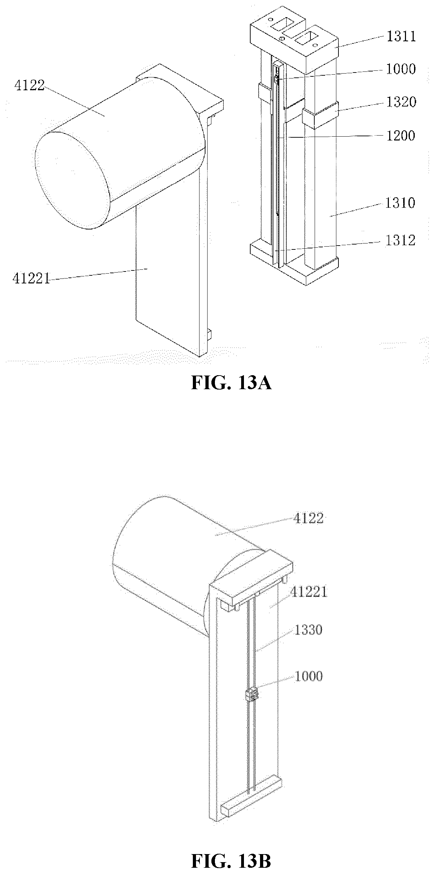

Description of reference numbers in FIG. 13A and FIG. 13B: 1310, switching frame; 1311, installation slot; 1312, puncture needle interface; 1320, switching frame driving mechanism; 1330, screw structure; 1200, puncture needle assembly; 1000, puncture needle driving device; 4122, slave hand; 41221, installation frame.

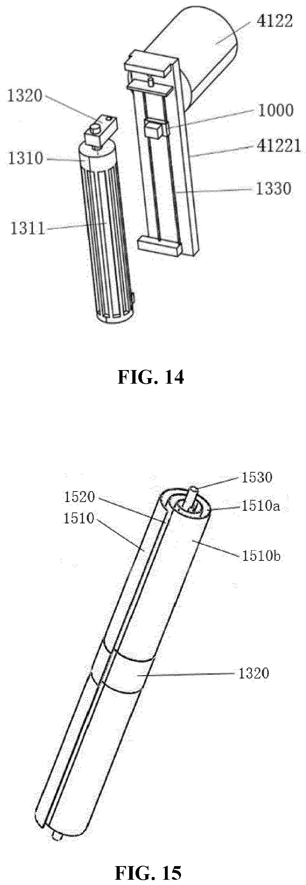

Description of reference numbers in FIG. 14: 1310, switching frame; 1320, switching frame driving mechanism; 1330, screw structure; 1000, puncture needle driving device; 1311, installation slot; 4122, slave hand.

Description of reference numbers in FIG. 15: 1510, installation surface; 1520, opening position; 1530, center position of spiral line; 1510a, first installation surface; 1510b, second installation surface.

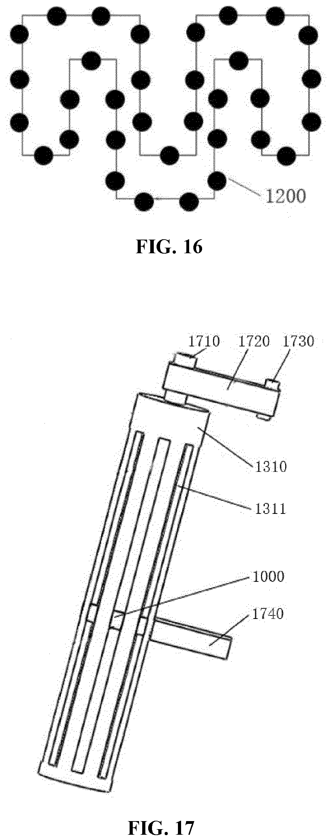

Description of reference numbers in FIG. 16: 1200, puncture needle assembly.

Description of reference numbers in FIG. 17 denote: 1710, rotating shaft; 1720, transmission unit; 1730, driving component; 1740, electrical interface; 1310, switching frame; 1311, installation slot; 1000, puncture needle driving assembly.

### DETAILED DESCRIPTION

In order to make the purpose, technical solutions, and advantages of the present disclosure clearer, the present disclosure is described and explained below in conjunction with the drawings and embodiments. It should be understood that specific embodiments described here are only used to explain the present disclosure, not to limit the present disclosure. Based on the embodiments provided in the present disclosure, all other embodiments obtained by those of ordinary skill in the art without any creative work shall fall within the scope of protection of the present disclosure. Additionally, it will be appreciated that, although such development efforts may be complex and lengthy, the technology disclosed in the present disclosure will be readily apparent to those of ordinary skill in the art to which the disclosure of the present disclosure relates. Some design, manufacturing, or production changes based on the content are only conventional technical means and should not be understood as insufficient disclosure of the content in the present disclosure.

Reference in the present disclosure to "an embodiment" means that a particular feature, structure, or characteristic described in connection with the embodiment may be included in at least one embodiment of the present disclosure. The occurrences of the phrase "an embodiment" in various places in the present disclosure are not necessarily all referring to the same embodiment, nor are separate or alternative embodiments mutually exclusive of other embodiments. It is explicitly and implicitly understood by those of ordinary skill in the art that the embodiments described in the present disclosure may be combined with other embodiments without conflict.

Unless otherwise defined, the technical terms or scientific terms involved in the present disclosure shall have the usual meanings understood by those with ordinary skills in the technical field to which the present disclosure belongs. Words such as "a", "an", "one" and "the" involved in the present disclosure do not indicate a limitation on quantity, and may indicate singular or plural. The terms "including", "comprising", "having" and any variations thereof mentioned in the present disclosure are intended to cover non-exclusive inclusion; for example, a process, method, system, product, or product that includes a series of steps or modules (units). The equipment is not limited to the listed steps or units, but may also include steps or units that are not listed, or may further include other steps or units inherent to these processes, methods, products, or equipment. Words such as "connected", "linked", "coupled" and similar words used in the present disclosure are not limited to physical or mechanical connections, but may include electrical connections, whether direct or indirect. "Multiple" referred to in the present disclosure means greater than or equal to two. "And/or" describes the relationship between related objects, indicating that three relationships can exist. For example, "A and/or B" can mean: A alone exists, A and B exist simultaneously, and B exists alone. The terms "first", "second", "third", etc. used in the present disclosure are only used to distinguish similar objects and do not represent a specific ordering of the objects.

An automated particle implantation system provided in some embodiments may implement radioactive particle implantation through an automated system. The automated particle implantation system includes an active system and a passive system. The passive system may communicate with the active system to realize that the active system controls the passive system and transmits force feedback information from the passive system to the active system. A communication process may be implemented based on mobile signals, wireless networks, or Bluetooth technology.

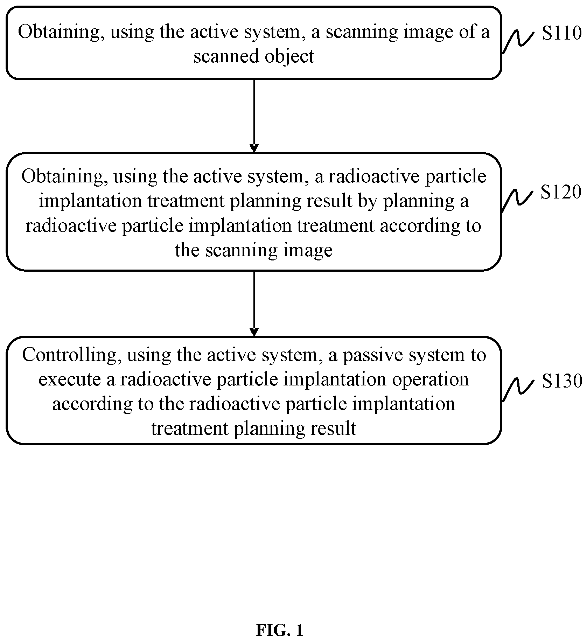

The automated particle implantation system may implement a radioactive particle implantation method when performing radioactive particle implantation. FIG. 1 is a flowchart illustrating an exemplary process of radioactive particle implantation according to some embodiments of the present disclosure. As shown in FIG. 1, the method includes the following steps.

In step S 110, the active system may obtain a scanning image of a scanned object.

During a process of the radioactive particle implantation, an implantation path needs to be planned. In order to plan the particle implantation path in real-time, in some embodiments, the scanning image of the scanned object may be introduced to obtain anatomical information of the scanned object in real-time.

In some embodiments, the active system may communicate with a scanning device. In the case of the radioactive particle implantation, the active system may send a control signal to the scanning device to control the scanning device to scan the scanned object in real-time. The scanning device may be a Positron Emission Computed Tomography (PET) system, a Computed Tomography (CT) system, a Magnetic Resonance Imaging (MRI) system. The scanning device may also be a multi- modality scanning system, such as PET-CT or PET-MR. Deformation of the tissue of the scanned object may affect the implantation of a particle. Therefore, a real-time scanning of the scanned object helps to correct a radioactive particle implantation treatment in real-time, so as to achieve a better treatment result. In some embodiments, after generating the scanning image of the scanned object, the scanning device pre-stores the scanning image in a storage device. The active system may communicate with the scanning device. When the radioactive particle implantation is required, the active system may obtain the scanning image of the scanned object from the storage device.

In some embodiments, the scanning device may be CT. CT uses accurately collimated X-ray beams, gamma rays, ultrasound, etc., together with extremely sensitive detectors, to perform cross-sectional scans one after another around a certain part of the scanned object. A scanning time of CT is short, and a scanning image obtained through CT may provide accurate anatomical positioning of the lesion, which is more conducive to implantation path planning. The scanned object may be a patient who needs the radioactive particle implantation.

In step S 120, the active system may obtain a radioactive particle implantation treatment planning result by planning a radioactive particle implantation treatment according to the scanning image.

In some embodiments, after obtaining the scanning image, the active system may identify and locate a region of interest based on anatomical information of the scanning image, wherein the region of interest may be a lesion that needs to receive puncture. Specifically, the active system may identify the region of interest through an image recognition algorithm, such as traditional machine learning or deep learning algorithms based on neural networks.

In some embodiments, after obtaining a position of the region of interest, the active system may obtain the radioactive particle implantation treatment planning result by planning the radioactive particle implantation treatment.

In some embodiments, the radioactive particle implantation treatment planning may include a target dose planning and a particle implantation path planning. In some embodiments, the radioactive treatment planning result includes a target dose determined through the target dose planning and a particle implantation path determined through the particle implantation path planning.

The target dose of the present disclosure refers to a radiation intensity of all radioactive particles that need to be implanted, as well as a count and a position of the radioactive particles. In some embodiments, the target dose may include a total radiation dose and/or a local radiation dose. The total radiation dose refers to a radiation dose composed of all radioactive particles received by the region of interest as a whole. The local radiation dose refers to a radiation dose composed of radioactive particles inserted at each target point position in the region of interest when multiple puncture implantations are required in the region of interest. The target point position refers to a position in the region of interest of the scanned object where the radioactive particles are to be implanted.

In some embodiments, the target dose may be determined based on a doctor's prescription. In some embodiments, the target dose planning needs to satisfy a dose planning standard, for example, that the normal human tissue other than the region of interest cannot be damaged.

In some embodiments, the total radiation dose is related to a size of the region of interest. In some embodiments, after determining the total radiation dose according to the doctor's prescription, the automated particle implantation system may set an initial value of the local radiation dose based on the total radiation dose and the size of the region of interest. For example, the initial value of the local radiation dose corresponding to each target point position may be obtained by dividing the total radiation dose by an area of the region of interest and then multiplying by an area of each target point position. As another example, the initial value of the local radiation dose for each target point position (i.e., an initial local radiation dose) may be obtained by dividing the total radiation dose by a count of target point positions in the region of interest.

In some embodiments, the initial local radiation dose may also be set or adjusted after the particle implantation path is obtained, so as to obtain a target value of the local radiation dose (i.e., a target local radiation dose). For example, after obtaining the particle implantation path, because the puncture needle assembly may be subject to some constraints and not arrive at some target point positions in the region of interest, it is necessary to remove a region corresponding to the some target point positions where the radioactive particles are not be implanted, reselect one or more target point positions in a remaining region of the region of interest and re-determine the target local radiation dose corresponding to each target point position.

In some embodiments, the implantation path may include a moving path that a puncture needle assembly moves from an initial position to a needle insertion position through a robotic arm of the automated particle implantation system. The robotic arm may refer to a slave hand in FIG. 4 below, and in some embodiments, and the slave hand is the robotic arm. In some embodiments, the particle implantation path may also include a puncture path for the radioactive particle implantation. Therefore, some embodiments also need to consider the needle insertion position during planning. The initial position refers to a position where the robotic arm of the automated particle implantation system is located when the scanned object and the automated particle implantation system are ready. The needle insertion position refers to a position of the robotic arm when the puncture needle assembly of the automated particle implantation system can directly perform a puncture action toward a corresponding target point position. The puncture path refers to a path along which a puncture needle in the puncture needle assembly at the needle insertion position travels from the beginning to extend to a corresponding target point position.

In some embodiments, the radioactive treatment planning result may include particle implantation path information. In some embodiments, the particle implantation path information may include a count of puncture needle assemblies, a needle insertion position of each puncture needle assembly, a target point position of each puncture needle assembly, and a count of particles at the target point position of each puncture needle assembly, and/or a moving path of the robotic arm.

In some embodiments, a preset motion constraint condition of the robotic arm may provide a reference for the implantation path planning. The motion constraint condition of the robotic arm refers to a constraint condition during the movement of the robotic arm. For example, the motion constraint condition of the robotic arm may include that the robotic arm moves from the initial position to the needle insertion position and there is no collision during the movement of the robotic arm, and the implantation path should avoid bones, human tissue, blood vessels, etc. In some embodiments, the implantation path may be jointly determined by the position of the region of interest, the total radiation dose, and/or the motion constraint condition of the robotic arm.

In step S130, the active system may control the passive system to execute a radioactive particle implantation operation according to the radioactive particle implantation treatment planning result.

In some embodiments, after the target dose planning and/or implantation path planning are completed, the active system may control the passive system to move according to the target dose and/or implantation path to complete the radioactive particle implantation operation.

In some embodiments, the automated particle implantation system may realize real-time the target dose planning and implantation path planning based on real-time scanning images through steps S110 to S130, thereby reducing operation time and improving operation efficiency. In traditional technologies, the target dose planning and the implantation path planning of the automated particle implantation system are independent of each other, which result in the automated particle implantation system being unable to execute a particle implantation path that satisfies a planned dose, or a particle implantation path executed by the automated particle implantation system may not satisfy a requirement of the target dose, so it takes a long time to adjust or correct. Moreover, traditional puncture processes are performed manually by a doctor, which are complex and time-consuming. In order to avoid radioactive attenuation, the radioactive particles may only be prepared after the planning is completed and before the operation. Therefore, during each operation, the radioactive particles may not be obtained in advance, and a patient needs to wait for the preparation of the radioactive particles, resulting in a prolonged surgical procedure. In the present disclosure, the scanning device is controlled by the active system to obtain real-time scanning images of the scanned object. At the same time, the active system may scientifically combine the target dose planning and particle implantation path planning according to the scanning images, thereby reducing the adjustment of the particle implantation path during the planning, solving the problems of the long cycle of the radioactive particle implantation, the cumbersome operation process, the high operation time cost and the low operation efficiency in traditional technologies. In some embodiments, the automated particle implantation system can realize automated implantation of the radioactive particles based on a combination of the active system and the passive system, thereby reducing operation time and improving operation efficiency. Therefore, multiple operations can be completed in a short period of time, and the radioactive particles can be prepared in advance without worrying about radioactive attenuation.

Implantation of the radioactive particles based on the active system and the passive system does not require the doctor to perform it manually, thereby reducing the dependence on the doctor's experience during the radioactive particle implantation, simplifying a surgical process, and further improving surgical efficiency.

When the scanning device is CT device, in traditional technologies, the scanned object needs to be scanned by the CT device first, and then the doctor performs a blind puncture based on the scanning image, and then the scanned object is scanned by CT device to verify the needle insertion position, repeat the process until all planned puncture needle punctures are completed, and finally, particle implantation is performed manually. Similarly, multiple CT scans are required during the implantation to confirm that positions of particle implantation are planned positions. In some embodiments of the present disclosure, the CT scanning and the radioactive particle implantation may be performed simultaneously on the scanned object the scanned object, thereby reducing a CT scan dose received by the scanned object during the radioactive particle implantation and improving the safety of the radioactive particle implantation.

In some embodiments, the active system may include a radiation planning module, and the radiation planning module may be configured to implement the target dose planning and/or implantation path planning to obtain the target dose (e.g., the total radiation dose and/or local radiation dose), and/ or the particle implantation path.

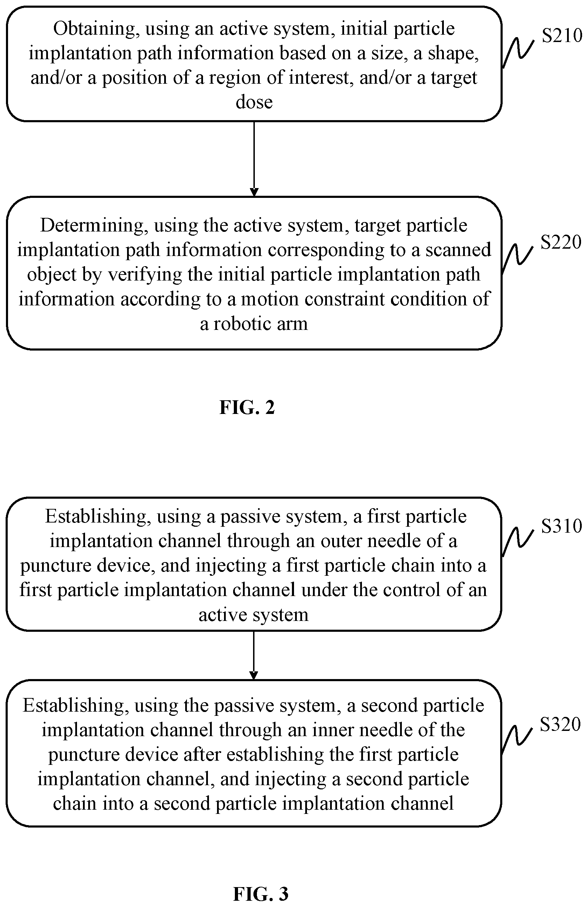

FIG. 2 is a flowchart illustrating an exemplary process of target dose and implantation path planning according to some embodiments of the present disclosure. As shown in FIG. 2, the method may include one or more of the following operations:

In operation S210, initial particle implantation path information may be obtained, by an active system, based on a size, shape, and/or position of a region of interest, and/or a target dose (e.g., a total radiation dose). The initial particle implantation path information may include an initial local radiation dose and/or an initial particle implantation path.

In some embodiments, the active system may determine particle implantation path information based on a scan image, target dose, and/or one or more motion constraint conditions of a robotic arm. In some embodiments, the active system may first determine the region of interest in the scan image, and then determine the particle implantation path information based on the region of interest, the target dose, and/or the motion constraint condition(s) of the robotic arm. In some embodiments, the active system may directly obtain a scan image in which the region of interest has been manually determined from a memory, and then determine the particle implantation path information based on the region of interest in the scan image, the target dose, and/or the motion constraint condition of the robotic arm. In some embodiments, the active system may determine the particle implantation path information based on the scan image, the target dose, the motion constraint condition of the robotic arm, and one or more additional conditions in a prescription issued by a doctor. The one or more additional conditions in the prescription issued by the doctor may refer to one or more pieces of guidance given by the doctor in the prescription based on the particular physical condition of a scanned object. For example, if there is a healing wound next to the region of interest, the doctor's guidance may be "to implant radioactive particles in the region of interest as far away from the healing wound as possible" while meeting requirements for the radiation treatment.

In some embodiments, the active system may obtain the initial particle implantation path information according to the size, shape, and/or position of the region of interest, and/or the target dose. In some embodiments, the active system may obtain the initial particle implantation path information according to one or more of the size, shape, position of the region of interest, the target dose, and a distribution of target point positions in the region of interest. In some embodiments, the distribution of target point positions in the region of interest may refer to whether target point positions are distributed on the edge or center of the region of interest, and whether they are concentrated or scattered in the region of interest.

In some embodiments, when determining an implantation path, the active system may first determine one or more initial radiation doses and an initial particle implantation path through TPS. The initial radiation dose(s) may include the total radiation dose and/or the initial local radiation dose. In some embodiments, when TPS is planning, after obtaining the total radiation dose, the initial local radiation dose may be determined at each needle insertion position as a reference. Specifically, an initial position of the robotic arm of a passive system may be a starting point of the initial particle implantation path, and an end point of the initial particle implantation path may be determined based on the position of the region of interest. The end point of the initial particle implantation path may be an initial target point position. The total radiation dose may be determined based on the size of the region of interest, and the initial local radiation dose may be determined based on a position of a target point position for puncturing the region of interest.

In operation S220, the initial particle implantation path information may be verified, by the active system, according to the motion constraint condition of the robotic arm to determine target particle implantation path information corresponding to the scanned object.

In some embodiments, the target particle implantation path information may include a target local radiation dose and/or a target particle implantation path.

In some embodiments, the active system may verify one or more pieces of information of the particle implantation path information.

In some embodiments, the initial particle implantation path may be inaccessible to the robotic arm or collision may occur as the robotic arm moves along the path given the motion constraint condition of the robotic arm. Therefore, after obtaining pieces of information including the total radiation dose, the initial local radiation dose, and the initial particle implantation path, it is necessary to verify the initial particle implantation path based on the motion constraint condition of the robotic arm, so that the target particle implantation path meets an accessibility requirement of the robotic arm. In some embodiments, punctures at different positions in the region of interest may affect the count of radioactive particles at that position, which may in turn affect an initial local radiation dose corresponding to the position. Therefore, while adjusting the initial particle implantation path, the initial local radiation dose also needs to be adjusted accordingly. Specifically, for the same region of interest, a plurality of punctures may be required to complete all the radioactive particle implantations. Therefore, for the plurality of punctures, different needle insertion positions or target point positions may correspond to different local radiation doses. For example, if the region of interest is spherical, a local radiation dose at the center of the sphere may be smaller than a local radiation dose at the edge, but a total radiation dose in the region of interest may not change. It should be noted that the radiation dose received at the center of the sphere is not only affected by the local radiation dose formed by radioactive particles positioned at the center of the sphere but also affected by radioactive particles positioned at the edge.

Through operations S210 and S220, the embodiment of the present disclosure is performed based on a radiation planning module in the active system. After obtaining the total radiation dose, the initial local radiation dose, and the initial particle implantation path, the initial particle implantation path may be verified directly based on the motion constraint condition of the robotic arm, and the target local radiation dose and the target particle implantation path may be obtained quickly. Compared with the problems existing in traditional technology, which are that, the total radiation dose, initial local radiation dose, and initial particle implantation path are determined through TPS in a planning department, and then the radiation plan is further adjusted in a surgery execution department, resulting in a longer time required for planning out the radiation procedure. The embodiment of the present disclosure unifies the motion constraint condition of the TPS and the robotic arm, which greatly reduces the determination time and improves surgical efficiency.

In some embodiments, when verifying the initial particle implantation path, in response to a determination that the verification passes, the active system determines the initial particle implantation path information as the target particle implantation path information corresponding to the scanned object; in response to a determination that the verification fails, the active system corrects the initial particle implantation information based on the region of interest, the target dose and/or the motion constraint condition of the robotic arm until the verification passes. In some embodiments, the initial particle implantation path information may include the initial local radiation dose and/or initial particle implantation path. In some embodiments, the active system may verify one or more pieces of the initial particle implantation path information.

In some embodiments, when verifying the initial local radiation dose and/or the initial particle implantation path in the initial particle implantation path information, it is first determined based on the motion constraint condition of the robotic arm whether the initial particle implantation path is inaccessible to the robotic arm and/or if there is a collision as the robotic arm moves. If the path is inaccessible or there is a collision, the verification fails, and the initial particle implantation path in the initial particle implantation path information needs to be adjusted. After adjusting the initial particle implantation path, the initial local radiation dose may be adjusted according to the target dose and a new needle insertion position in the region of interest, and/or target point position, until the target local radiation dose and target particle implantation path are obtained. In some embodiments, the initial particle implantation path may be a moving path of the robotic arm. The motion constraint condition of the robotic arm may include but not limited to: that the robotic arm is able to reach a needle insertion position, that there is no collision as the robotic arm moves, and that a puncture path for radioactive particle implantation after reaching the needle insertion position does not pass through bones, dangerous tissues, organs, blood vessels, etc. In some embodiments, a planned total radiation dose and local radiation dose must meet a prescribed dose, and the local radiation dose must not exceed a standard, and at the same time comply with the Dose Volume Histogram (DVH) required for radiation.

In some embodiments, when a planned initial particle implantation path needs to be adjusted, an operator may fine-tune the initial particle implantation path at a console of the active system, or the active system may gradually adjust the path based on the motion constraint condition of the robotic arm until the target particle implantation path information meets the motion constraint condition of the robotic arm. In some embodiments, the initial particle implantation path and the initial local radiation dose may be verified and adjusted according to the region of interest and the motion constraint condition of the robotic arm, so as to obtain the target local radiation dose and the target particle implantation path, thereby improving the surgical effect.

In other embodiments, after a radioactive particle implantation surgery is completed, a result of the radioactive particle implantation may need to be verified for further requirements, for example, whether a radiation dose received at each target point position in the region of interest reaches a prescribed dose. The radiation dose received at a target point position may equal to the local radiation dose corresponding to the current target point position, adding the radioactive impact of a local radiation dose at other target point positions on the current target point position. In some embodiments, before the radioactive particle implantation, the doctor may give the prescribed dose as a target dose, and when the TPS plans the radioactive particle implantation procedure, the prescribed dose may be used as one of the constraints for the radiation dose determination. For example, one criterium for successful radioactive particle implantation is that more than 95% of the region of interest receives a radiation dose that reaches the prescribed dose.

During the radioactive particle implantation, ideally, the result achieved by implantation should be the same as the original plan. However, due to a patient's breathing and other external factors, the implantation may deviate from a planned process. Therefore, after completing the radioactive particle implantation, it is necessary to verify again to confirm whether more than 95% of the region of interest receives the radiation dose that reaches the prescribed dose, so as to evaluate the effect of the procedure. In some embodiments, after all puncture needle assemblies in a radioactive particle implantation treatment planning result have finished particle implantation, the active system may control a scanning device to scan the scanned object to test the result of the implantation, thereby evaluating the effect of the procedure.

In some embodiments, the passive system may further include a puncture device, wherein the puncture device includes a puncture device terminal and at least one puncture needle assembly mounted on the puncture device terminal. A size and shape of the puncture device terminal may relate to the number of the at least one puncture needle assembly in the puncture device terminal. Each of the at least one puncture needle assembly may be used to establish a particle implantation channel. The particle implantation channel in some embodiments may be established according to a puncture path in a pre-determined particle implantation path. In some embodiments, the passive system may be configured to execute a radioactive particle implantation operation according to a radioactive particle implantation planning result. Specifically, a slave hand of the passive system may move the robotic arm to a planned needle insertion position under the control of the active system, cause the puncture needle assembly to be equipped with the needle and reach the target point position, establishing the particle implantation channel. The slave hand of the passive system may implant the radioactive particles via the particle implantation channel, that is, implanting the radioactive particles at the target point position in the region of interest in the form of particle chain and withdrawing the puncture needle assembly afterwards. Then, the slave hand of the passive system may move the robotic arm to a next needle insertion position and implanting one or more particles using a next puncture needle assembly, until all puncture needle assemblies in the radioactive particle implantation treatment plan have finished radioactive particle implantation.

In some embodiments, the size and shape of the puncture device terminal may correspond to a count of puncture needle assemblies in the puncture device, that is, in the case of a plurality of puncture needle assemblies, the puncture device terminal may have a plurality of sizes and shapes to accommodate different regions of interest. Specifically, there may be two types of different regions of interest. One is that there are regions of interest in different parts of the same surgery, and the other is that there are regions of interest in different parts of different surgeries. If there are regions of interest in different parts, the count of puncture needle assemblies may be usually determined based on a planning requirement. In some embodiments, since the count of puncture needle assemblies required for the region of interest varies from 2 to 40, if a design of the puncture device terminal is fixed, then the count of puncture needle assemblies may only be determined based on a maximum count of puncture needle assemblies. Therefore, a fixed design may unnecessarily increase the size of the puncture device terminal and cause waste in most clinical scenarios. Therefore, in some embodiments, based on the count of radioactive particles of the regions of interest in different parts, the size and shape of the puncture device terminal may be adjusted to adapt to the count of different puncture needle assemblies, so as to save surgical costs.

In some embodiments, the radiation planning module of the active system may be further configured to determine the count of radioactive particles according to the target dose (e.g., the local radiation dose), and then the passive system may obtain the count of radioactive particles through communication. The count of radioactive particles may be calculated based on the target dose (e.g., the local radiation dose) and a radioactivity of a single radioactive particle. In some embodiments, the radioactive particle may be iodine 125. In some embodiments, the passive system may include a particle chain generating mechanism. The particle chain generating mechanism may be used to form the particle chain in the particle implantation channel according to the count of radioactive particles and a filler material during the radioactive particle implantation. The filler material may play a role of space-filling during the formation of the particle chain, specifically, while implanting the radioactive particles into the region of interest, a space between two radioactive particles may be filled with the filler material. The filler material in some embodiments may be a biocompatible and degradable material, such as polycaprolactone. In some embodiments, the particle chain may be in any one of a straight line, an arc shape, a ring shape, a helical shape, a bifurcated shape, a cross shape, a mesh structure, or a combination structure of any count of particle chains. In some embodiments, radioactive particles that need to be implanted do not need to be positioned in advance. The particle chain may be generated in real-time by the particle chain generating mechanism during the operation according to the planned target dose and implantation path and may be implanted into the target point position in the region of interest through the particle implantation channel at one time. The above-mentioned method can greatly improve the operation efficiency and shorten the operation time, allowing doctors to complete procedures on multiple patients with similar medical conditions in one day. On the other hand, the automated particle implantation system can automatically count radioactive particles implanted in each patient to avoid errors, as well as being able to support preoperative reservation of particles, which allows the hospital to order large counts of radioactive particles in advance for use, saving the ordering time.

In some embodiments, the particle chain generating mechanism may include a first material clamp and a second material clamp. In some embodiments, the radioactive particles may be stored in the first material clamp, and the filler material may be stored in the second material clamp. The first material clamp and the second material clamp may be both positioned at an end of the robotic arm of the passive system. When particle chain implantation is required, the robotic arm may obtain the radioactive particles and filler material from the first material clamp and the second material clamp respectively and may implant the radioactive particles and filler material sequentially into the region of interest through the puncture device. Usually, the number of radioactive particles in the first material clamp may be for more than one patient, so as to meet the needs of consecutive surgeries of the radioactive particle implantation.

FIG. 3 is a flowchart illustrating an exemplary process of radioactive particle implantation according to some embodiments of the present disclosure. As shown in FIG. 3, the method includes following steps.

In step S310, the passive system may establish a first particle implantation channel through an outer needle of a puncture device, and inject a first particle chain into the first particle implantation channel under the control of an active system;

In step S320, the passive system may establish a second particle implantation channel through an inner needle of the puncture device after establishing the first particle implantation channel, and inject a second particle chain into the second particle implantation channel.

In some embodiments, a puncture device terminal is a multi-needle terminal, that is, the puncture device includes a plurality of puncture needle assemblies with the same or different sizes and/or shapes, and only one puncture needle assembly is used for each implantation. After the puncture of one puncture needle assembly and the implantation of the corresponding radioactive particles are completed, pull out a puncture needle of the puncture needle assembly, switch to a next puncture needle assembly to establish a new particle implantation channel, and repeat the process until all radioactive particle implantation channels are established and the implantation of the radioactive particles are completed.

In some embodiments, the puncture needle in a puncture needle assembly includes an inner needle and an outer needle. After the entire puncture needle punctures into the body of a scanned object, the inner needle protrudes, and a particle implantation channel is formed inside the outer needle. A particle chain arrives at a region of interest through the particle implantation channel under the guidance of a robotic arm of the passive system. In some embodiments, the inner needle may have a solid structure and fill a cavity of the outer needle, so to prevent air from entering the cavity of the outer needle during a process of establishing the particle implantation channel.

Through steps S310 to S320, the puncture device in some embodiments may complete a plurality of puncture tasks in one operation, thereby improving surgical efficiency.

In some embodiments, if a position of the scanned object changes or the scanned object breathes too fast, a planned target dose and a particle implantation path need to be adjusted. Therefore, in some embodiments, a pose of the scanned object needs to be detected in real-time, so to avoid a failure of the planned target dose and particle implantation path due to the motion of the scanned object. Specifically, a body surface position of the scanned object through a scanning device may be obtained. In some embodiments, the body surface position is a body surface position corresponding to the region of interest, and the body surface position may be obtained by an imaging device installed on an inner wall of the scanning device. For example, the imaging device may perform a body surface imaging on the scanned object. After the body surface imaging is performed, the active system may perform a calculation according to the body surface position. In response to a determination that a change amount of the body surface position within a preset time is greater than or equal to a preset change threshold, it is considered that the motion of the scanned object has affected a planned target dose and implantation path. At this time, the active system modifies the planned target dose and implantation path based on a current scanning image, and obtains a new target dose and implantation path, thereby reducing surgical cost and improving surgical effect.

In some embodiments, the preset time and the preset change threshold may be set based on a doctor's experience, or may be simulated based on an algorithm. In some embodiments, when a displacement of the scanned object is too large, the doctor may also complete an operation and control of the passive system through the active system based on his own experience. For example, if the body of the scanned subject moves hugely and the displacement is too large, the doctor may judge based on experience that the change volume of the body surface position within the preset time is obviously greater than the preset change threshold, thereby stopping an operation of the passive system through the active system.

In other embodiments, the position information of the region of interest in the scanning image may also be obtained in real-time. When a change of the position information of the region of interest is greater than or equal to a preset position change threshold of the region of interest, it is also necessary to adjust and correct the target dose and implantation path. Specifically, an operator may fine-tune the implantation path at a console of the active system, or the active system may gradually adjust the implantation path according to a motion constraint condition of a robotic arm.

In some embodiments, the active system includes a display device. The display device is configured to display at least one of the scanning image, the radioactive particle implantation treatment planning result (e.g., the particle implantation path), a remaining amount of radioactive particles to be implanted, or a motion state of the passive system. Specifically, the display of the motion state of the passive system includes displaying an operating state and operating steps, prompting the operator for the next operation, and displaying a scanning image of the radioactive particles in real-time during particle implantation, so to guide the operator to complete the particle implantation. The remaining amount of radioactive particles to be implanted may assist in planning of a plurality of radioactive particle implantation surgeries. For example, if the remaining amount of radioactive particles to be implanted is sufficient, a radioactive particle implantation surgery may be directly performed on the next scanned object. This embodiment is based on the display device, which may provide an image of part or the entire process of the radioactive particle implantation, making a radioactive particle implantation surgery visible and reducing the difficulty of the surgery.

In some embodiments, before the scanning device obtains the scanning image of the scanned object, it is also necessary to register a coordinate system of the passive system with a coordinate system of the scanning device, so as to align positions of the region of interest in the scanning image and a corresponding region of interest in the actual scanned object. Specifically, first coordinates of a marker or other specific objects in the coordinate system of the passive system and second coordinates of the marker or the other specific objects in the coordinate system of the scanning device may be obtained. A position and space relationship between the coordinate system of the passive system and the coordinate system of the scanning device may be established based on the first coordinates and the second coordinates. A transformation matrix may be calculated and the registration may be completed. In some embodiments, the registration ways may include a magnetic registration (i.e., a functional magnetic resonance image registration), an optical registration (e.g., an infrared image registration and/or a visible light image registration), a physical position registration, etc.

The present disclosure further provides another automated particle implantation robotic system 40 for radioactive particle implantation. FIG. 4 is a schematic diagram illustrating an exemplary structure of the automated particle implantation robotic system 40 according to some embodiments of the present disclosure. As shown in FIG. 4, the automated particle implantation robotic system 40 includes an automated particle implantation system 41 and a scanning device 42. In some embodiments, the automated particle implantation system 41 includes an active system 411 and a passive system 412. The active system 411 includes a first processor 4111 and a master hand 4112. The passive system 412 includes a slave hand 4121. In some embodiments, the slave hand 4121 is mechanically connected to a puncture device, and the scanning device 42 is communicatively connected to the first processor 4111. The first processor 4111 may control the scanning device 42 to obtain a scanning image of a scanned object. Further, the first processor 4111 determines a target dose corresponding to the scanned object according to the region of interest in the scanning image, and determines a particle implantation path corresponding to scanned object according to the region of interest in the scanning image and a preset motion constraint condition of a robotic arm. The master hand 4112 controls the passive hand 4121 to move to implant a target dose of radioactive particles according to the particle implantation path. In some embodiments, the master hand 4112 may be the robotic arm. In some embodiments, the slave hand 4121 may be a different robotic arm than the master hand 4112.

In some embodiments, the automated particle implantation robotic system 40 may realize real-time target dose and particle implantation path planning based on the scanning image, thereby reducing operation time and improving operation efficiency.

In traditional technologies, the target dose planning and the implantation path planning of the automated particle implantation system are independent of each other, which may result in the automated particle implantation system being unable to execute a particle implantation path that satisfies a planned dose, or a particle implantation path executed by the automated particle implantation system does not satisfy a target dose, so it takes a long time to adjust or correct. Moreover, a puncture process needs to be completed manually by a doctor, which is complex and time-consuming. Furthermore, radioactive particles may only be prepared after the planning is completed and before an operation to avoid radioactive attenuation of the radioactive particles. Therefore, the radioactive particles may not be obtained in advance. During each operation, a patient needs to wait for the preparation of radioactive particles, resulting in prolonged surgical procedures.

In some embodiments of the present disclosure, the active system 411 controls the scanning device 42 to obtain a real-time scanning image of the scanned object, and further scientifically combines the radiation dose planning and implantation path planning based on the scanning image, thereby reducing the adjusting time on the implantation path during the planning. In some embodiments, the automated particle implantation system 41 in the present disclosure realizes automated implantation of radioactive particles based on a combination of the active system 411 and the passive system 412, which reduces operation time, improve operation efficiency, and complete multiple surgeries in a short time. Therefore, the radioactive particles may be prepared in advance without worrying about the radioactive attenuation of the radioactive particles.

In some embodiments, the passive system 412 may further include a second processor 4122. The first processor 4111 and the second processor 4122 are communicatively connected, and the second processor 4122 is connected to the slave hand 4121 to realize an interaction between the active system 411 and the passive system 4122. In some embodiments, the second processor 4122 may also control a particle chain and a multi-needle terminal. Based on a communication connection between the second processor 4121 and the first processor 4111, the second processor 4122 controls the slave hand 4121 to establish a particle implantation channel through the puncture device according to the target dose and the particle implantation path.

In some embodiments, the automated particle implantation system may be the automated particle implantation system 41 for radioactive particle implantation in any of the above embodiments.

In some embodiments, the passive system 412 may further include the puncture device. In some embodiments, the puncture device has a puncture device terminal and at least one puncture needle assembly mounted on the puncture device terminal. A size and/or a shape of the puncture device terminal is related to a count of puncture needle assemblies in the puncture device. A puncture needle assembly is used to establish the particle implantation channel.

In some embodiments, the passive system 412 may include a particle chain generating mechanism, which is used to form the particle chain in the particle implantation channel according to a count of radioactive particles and a space-occupying material during the radioactive particle implantation.

In some embodiments, the active system 411 may include a display device. The display device is used to display at least one of the scanning image, a radioactive particle implantation treatment planning result, a remaining amount of radioactive particles to be implanted, or a motion state of the passive system 412.

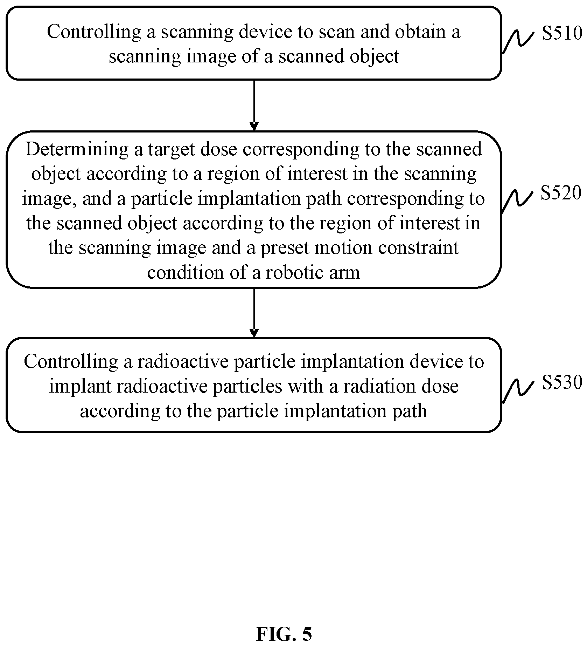

The present disclosure further provides a radioactive particle implantation method. FIG. 5 is a flowchart illustrating another process of radioactive particle implantation according to some embodiments of the present disclosure. As shown in FIG. 5, the method includes the following steps.

In step S510, a scanning device may be controlled to scan and obtain a scanning image of a scanned object.

In step S520, a target dose corresponding to the scanned object may be determined according to a region of interest in the scanning image, and a particle implantation path corresponding to the scanned object may be determined according to the region of interest in the scanning image and a preset motion constraint condition of a robotic arm.

In step S530, a radioactive particle implantation device may be controlled to implant radioactive particles with a radiation dose according to the particle implantation path.

The radioactive particle implantation method in some embodiments may be applied to an automated particle implantation system, where an active system of the automated particle implantation system controls the scanning device to scan, so as to achieve target dose and particle implantation path planning, and controls the particle implantation device to complete particle implantation. Specifically, the radioactive particle implantation device in some embodiments may be a passive system of the automated particle implantation system.

In traditional technologies, the target dose planning and the implantation path planning of the automated particle implantation system are independent of each other, which will result in the automated particle implantation system being unable to execute the particle implantation path that satisfies a planned dose, or a particle implantation path executed by the automated particle implantation system does not satisfy a target dose, so it takes a long time to adjust or correct. Moreover, a puncture process needs to be completed manually by a doctor, which is complex and time-consuming. Furthermore, radioactive particles may only be prepared after the planning is completed and before an operation, so as to avoid radioactive attenuation of the radioactive particles. Therefore, the radioactive particles may not be obtained in advance. During each operation, patients all need to wait for the preparation of radioactive particles, resulting in prolonged surgical procedures.

Through steps S510 to S530, in the present disclosure, the active system controls the scanning device to obtain the scanning image of the scanned object in real time. At the same time, the active system scientifically combines the target dose planning and the implantation path planning according to the scanning image, which solves the problems of a long implantation cycle and cumbersome surgical procedures, resulting in a high cost of surgical time and low surgical efficiency, thereby reducing the time for adjusting the particle implantation path during the planning and improving surgical efficiency.

The radioactive particle implantation method in some embodiments may be applied to the automated particle implantation system for the radioactive particle implantation or the automated particle implantation robotic system for the radioactive particle implantation in any of the above embodiments.

In some embodiments, the automated particle implantation system includes the active system and the passive system. The passive system is communicatively connected with the active system, and the radioactive particle implantation method is controlled by the active system. The active system also verifies the target dose and implantation path.

In some embodiments, the passive system further includes a puncture device, wherein the puncture device has a puncture device terminal, and a size and shape of the puncture device terminal correspond to a count of puncture needle assemblies in the puncture device. A puncture needle assembly is used to establish a particle implantation channel.

In some embodiments, the passive system includes a particle chain generating mechanism. The particle chain generating mechanism is configured to generate a particle chain in the particle implantation channel according to a count of radioactive particles and a space-occupying material during the radioactive particle implantation.

In some embodiments, the active system includes a display device. The display device is configured to display at least one of the scanning image, the particle implantation path, a remaining amount of radioactive particles to be implanted, or a motion state of the passive system.

In some embodiments, the automated particle implantation robotic system may include an automated particle implantation system and a scanning device. The automated particle implantation system may include an active system and a passive system. The active system may include a first processor and a master hand, the passive system may include a slave hand, the slave hand is mechanically connected to a puncture device, and the scanning device is communicatively connected to the first processor.

It should be noted that the steps shown in the above process or the flowchart of the accompanying drawings may be executed in a computer system such as a set of computerexecutable instructions, and although a logical sequence is shown in the flowchart, in some cases, the steps shown or described may be performed in an order different from that herein.

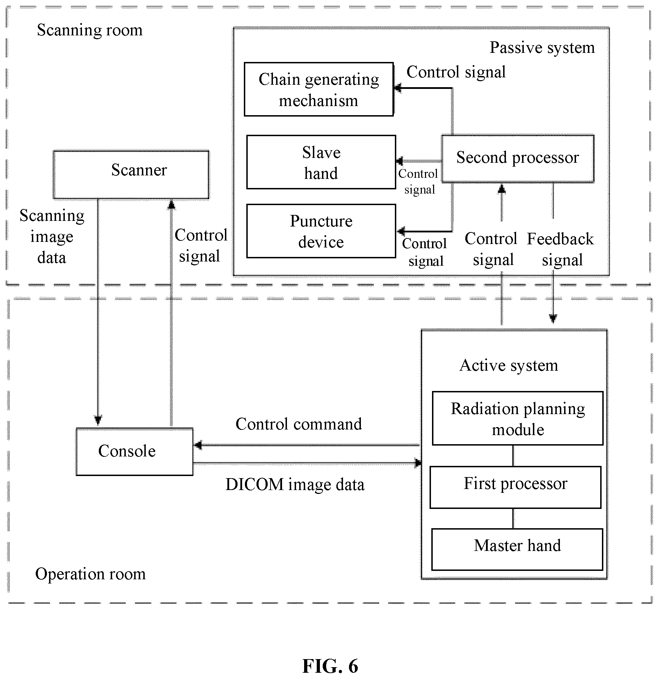

FIG. 6 is a schematic diagram illustrating an exemplary structure of an automated particle implantation system according to some embodiments of the present disclosure. As shown in FIG. 6, the automated particle implantation system may be distributed between an operation room and a scanning room. Specifically, the automated particle implantation system includes a scanning device, an active system, and a passive system.

In some embodiments, the scanning device includes a scanner and a console. The scanner includes a scanning bed and a rack. The scanning bed and the rack are located in the scanning room, and the console is located in the operation room.

In some embodiments, the active system is located in the operation room and includes a master hand, a radiation planning module, and a first processor. The master hand is used to allow an operator in the operation room to remotely control a slave hand in the scanning room, so that under the guidance of real-time scanning images, a multi-needle terminal is transported to a planned target point position and the establishment of a particle implantation channel is completed. The radiation planning module includes a TPS and a robot path planning unit for realizing an interactive verification and planning of radiation dose and implantation path. The first processor is used to communicate with the scanning device and the passive system, respectively, so as to obtain a scanning image from the console and control the scanner and the passive system.

In some embodiments, the passive system is located in the scanning room. The passive system includes a second processor, a particle chain generating mechanism, the slave hand, and a puncture device. Specifically, the second processor is used to communicate with the first processor, execute a control command from the active system, and transmit a force feedback signal of a resistance suffered by the slave hand to the master hand during a puncture process. At the same time, the second processor is also used to transmit a control signal to the particle chain generating mechanism, the slave hand, and the puncture device, so that the particle chain generating mechanism completes the production of the particle chain, causes the slave hand move according to a particle implantation path, causes the puncture device perform a puncture to complete the establishment of the particle chain. The slave hand in some embodiments is composed of a plurality of robotic arms, and the puncture device has a multi-needle terminal.

The automated particle implantation system in some embodiments implements following steps when performing radioactive particle implantation.

In step S1, the passive system may be registered with the scanning device. In some embodiments, the scanning device is CT.

In step S2, the first processor in the active system may obtain basic information about a scanned object, such as a height, a weight, a gender, a past medical history, and other related information, and then controls the scanning device to scan the scanned object to obtain a scanning image, and further identifies a region of interest based on the scanning image. In some embodiments, the scanning image is transmitted to the active system through the console of the scanning device, and a format of the scanning image is Digital Imaging and Communications in Medicine (DICOM).

In step S3, the radiation planning module in the active system may plan a total radiation dose, a local radiation dose, and a particle implantation path. Specifically, the radiation planning module verifies and corrects a total radiation dose, an initial local radiation dose, and an initial particle implantation path obtained based on the TPS until a target local radiation dose and a target particle implantation path that satisfy a dose planning criteria and a motion constraint condition of a robotic arm are obtained.

In step S4, the passive system may obtain the target local radiation dose and the target particle implantation path through the second processor, and installs the multi-needle terminal and radioactive particles at an end of the slave hand. A count of radioactive particles is determined according to the local radiation dose.

In step S5, after an aseptic arrangement is perform on an operating room and an anesthesia is performed on the scanned object, the slave hand may move to a target point position determined by the target particle implantation path under the control of the master hand.

In step S6, the multi-needle terminal may establish a particle implantation channel through a puncture needle assembly, place a particle chain in the particle implantation channel, and then pulls out a puncture needle of the puncture needle assembly. The particle chain is generated by the particle chain generating mechanism according to the radioactive particles and a degradable space-occupying material.

In step S7, the multi-needle terminal may switch to the next puncture needle assembly, the slave hand moves to the next needle insertion position until all puncture tasks are completed; and

In step S8, the implantation effect of the radioactive particles may be confirmed based on a scanning image. After the implantation is completed, the active system and the passive system are reset to complete an operation.

In some embodiments, before the scanning device obtains the scanning images of the scanned object, it is also necessary to register a coordinate system of the passive system with a coordinate system of the scanning device to achieve an alignment between the region of interest in the scanning image and a region of interest in the actual scanned object. Specifically, a registration method includes the following operations. First, first coordinates of a marker or other featured objects in the coordinate system of the passive system and second coordinates of the marker and other featured objects in the coordinate system of the scanning device may be obtained, and a position and space relationship between the coordinate system of the passive system and the coordinate system of the scanning device may be established based on the first coordinates and the second coordinates, and then a transformation matrix may be calculated to complete the registration. Ways to implement the registration include a functional magnetic resonance image registration, an infrared image registration, a visible light image registration, and/or a physical position registration, etc.

In some embodiments, the total radiation dose may be determined based on a radiation dose prescribed by a doctor for the scanned object.

The automated particle implantation system in some embodiments may perform real-time local radiation dose and particle implantation path planning for the region of interest under the real-time guidance of the scanning images, and realize automatic implantation of the radioactive particles at the same time. Based on a combination of the scanning image, active system, and passive system, an automated operation of the radioactive particle implantation is realized, which greatly reduces the dependence on the doctor's experience. Moreover, based on the real-time scanning images, the implantation effect of radioactive particles can be improved.

Due to the automated operation, the automated particle implantation system can shorten a patient's waiting time, reduce the patient's cost and time consumption, and at the same time improve a matching degree between a final implantation result and an expected result.

The automated particle implantation system in the present disclosure also reduces the operation time of a single operation and a count of required medical staff, and improves the efficiency of the operation. Based on the cooperation between the master hand and the slave hand, the particles can smoothly reach a planned target point position, greatly improving the accuracy of particle implantation.

It should be noted that each of the above-mentioned modules may be a function module or a program module, and may be realized by software or by hardware. For the modules implemented by hardware, the above modules may be located in the same processor; or the above modules may be located in different processors in any combination.

The method provided in the present disclosure may be executed in a terminal, computer, or similar computing device. Taking operating on a terminal as an example, FIG. 7 is a schematic diagram illustrating an exemplary hardware structure of a terminal for a radioactive particle implantation method according to some embodiments of the present disclosure. As shown in FIG. 7, a terminal 70 may include one or more (only one is shown in FIG. 7) processors 702 and a memory 704 for storing data. The processor 702 may include but is not limited to a processing device such as a microprocessor MCU or a programmable logic device FPGA. Optionally, the terminal may further include a transmission device 706 and an input and output device 708 for communication. Those skilled in the art may understand the structure shown in FIG. 7 is only for illustration and does not limit the structure of the above-mentioned terminal. For example, the terminal 70 may also include more or fewer components than those shown in FIG. 7, or have a different configuration than that shown in FIG. 7.

The memory 704 may be used to store a control program, for example, software programs and modules of application software, such as a control program corresponding to the radioactive particle implantation method in the embodiment of the present disclosure. The processor 702 executes various functional applications and data processing by operating the control program stored in the memory 704, that is, implementing the above methods. The memory 704 may include high-speed random-access memory, and may also include a non-volatile memory, such as a magnetic storage device, a flash memory, or other non-volatile solidstate memory. In some examples, the memory 704 may further include a memory located remotely relative to the processor 702, and the remote memory may be connected to terminal 70 via a network. Examples of the above-mentioned networks include but are not limited to the Internet, an intranet, a local area network, a mobile communication network, or combinations thereof.

The transmission device 706 is used to receive or send data via a network. The above-mentioned specific examples of the network may include a wireless network provided by a communication provider of the terminal 70. In one example, the transmission device 706 includes a network interface controller (NIC), which may be connected to other network devices through a base station to communicate with the Internet. In one embodiment, the transmission device 706 may be a radio frequency (RF) module, which is used to communicate with the Internet wirelessly.

This embodiment also provides an electronic device, including a memory and a processor. A computer program is stored in the memory, and the processor is configured to operate the computer program to perform the steps in any of the above method embodiments.

Optionally, the above-mentioned electronic device may further include a transmission device and an input and output device, wherein the transmission device is connected to the above-mentioned processor, and the input and output device is connected to the above-mentioned processor.

Optionally, in some embodiments, the above-mentioned processor may be configured to execute following steps through a computer program.

In step Y1, an active system may obtain a scanning image of a scanned object by controlling a scanning device to perform a scan on the scanned subject.

In step Y2, the active system may determine a target dose corresponding to the scanned object based on a region of interest in the scanning image, and determine a particle implantation path corresponding to the scanned object based on the region of interest in the scanning image and a preset motion constraint condition of a robotic arm.

In step Y3, the active system may control a passive system to move, so as to implant a target dose of radioactive particles according to the particle implantation path.

In combination with the radioactive particle implantation method in the foregoing embodiments, the embodiments of the present disclosure may provide a storage medium for implementation. The storage medium stores a computer program. When the computer program is executed by the processor, any one of the radioactive particle implantation methods in the above embodiments is implemented.

In the existing puncture needle implantation surgery, a count of implanted radioactive particles of radioactive particle chains used to treat lesions usually is not adjusted according to the situation of an individual patient, so the count of implanted radioactive particles during the operation may not be accurately controlled according to the actual situation of individual patient. Based on this, it is necessary to provide a particle chain generating mechanism and a puncture device terminal.

Please refer to FIG. 8, FIG. 9A, and FIG. 9B. FIG. 8 is a schematic diagram illustrating an exemplary structure of a particle chain generating mechanism 800 according to some embodiments of the present disclosure. FIG. 9A is a schematic diagram illustrating an exemplary structure of a first material clap 811 or a second material clap 812 in a particle chain generating mechanism according to some embodiments of the present disclosure. FIG. 9B is a schematic diagram illustrating an exemplary structure of a particle chain 813 generated by the particle chain generating mechanism 800 shown in FIG. 8.

Some embodiments of the present disclosure provide the particle chain generating mechanism 800, which is applied to a terminal execution device of a surgical robot, especially to a puncture needle driving device, so that the particle chain generating mechanism 800 may input specific therapeutic drugs (e.g., the particle chain 813), etc. to a target part (e.g., a lesion).

The particle chain generating mechanism 800 is used to implant the particle chain 813 into a puncture needle assembly through a trocar needle driving mechanism. The particle chain generating mechanism 800 includes a storage unit 810 and an implanting assembly 820. In some embodiments, at least a portion of the implanting assembly 820 is inserted in the storage unit 810 and can selectively cause one or more materials located in the storage unit 810 to form the particle chain 813 in an orderly manner and implant the particle chain 813 into the puncture needle assembly. The implanting assembly 820 is partially installed in the storage unit 810 and outputs the particle chain 813 in the storage unit 810. The storage unit 810 is used to store at least two materials for generating the particle chain 813. The implanting assembly 820 is used to output the materials in the storage unit 810. The materials may include a radioactive particle 813a and a space-occupying material 813b. The implanting assembly 820 may selectively output the materials in the storage unit 810 in an orderly manner according to a preset purpose. For example, a ratio between the radioactive particle 813a and the space-occupying material 813b, and an arrangement order of the two may all be formed according to the preset purpose (as shown in FIG. 9B ). With this arrangement, the particle chain generating mechanism 800 may form the particle chain 813 in an orderly manner, and the particle chain generating mechanism 800 may input the particle chain 813 into a target part according to a preset or real-time setting during an operation of a surgical robot.

Specifically, the storage unit 810 and the implanting assembly 820 may move relative to each other, so that the radioactive particle 813a and the space-occupying material 813b stored at different positions in the storage unit 810 may be selectively output under an action of the implanting assembly 820, and form the particle chain 813 in an orderly manner. A relative movement between the implanting assembly 820 and the storage unit 810 may be in the form of translation or rotation, so that a relative position between the implanting assembly 820 and the storage unit 810 may be changed, and the implanting assembly 820 may output different materials correspondingly. In some embodiments, the storage unit 810 may adopt a clip-type storage structure, and the implanting assembly 820 may adopt a push rod 822 as an output structure, which may be set accordingly according to actual needs.

In some embodiments, the storage unit 810 includes a first material clamp 811 and a second material clamp 812. The first material clamp 811 is used to store the radioactive particle 813a, and the second material clamp 812 is used to store the space-occupying material 813b which is degradable in the human body. In some embodiments, the first material clamp 811 and the second material clamp 812 may be stacked one above the other or arranged side by side, and specifically, may be set up according to a relative movement direction and movement way between the implanting assembly 820 and the storage unit 810. The implanting assembly 820 may be selectively connected to the first material clamp 811 or the second material clamp 812 through the relative movement with the storage unit 810, so as to orderly use the radioactive particle 813a in the first material clamp 811 or the space-occupying material in the second material clamp 813 to generate the particle chain 813.

In some embodiments, the first material clamp 811 and/or the second material clamp 812 may have a structure similar to a magazine clamp, wherein a structure of the second material clamp 812 is as shown in FIG. 9A. A material-discharging opening of the second material clamp 812 is provided with a position-limiting part 8121, and an elastic piece (not shown in FIG. 9A) is provided inside the second material clamp 812 to push the space-occupying material 813b therein toward the material-discharging material. In some embodiments, the elastic piece may be a spring structure, and the position-limiting part 8121 is used to limit the space-occupying material 813b in the second material clamp 812 to the material-discharging opening without completely detaching from the second material clamp 812. A structure of the first material clamp 811 may be the same as or similar to the structure of the second material clamp 812.

It can be understood that in some alternative embodiments, the storage unit 810 may also include three or more material clamps, and correspondingly store three or more materials for generating the particle chain 813.

In some embodiments, at least a portion of the implanting assembly 820 is rotatably connected to the storage unit 810, so as to be selectively connected to the first material clamp 811 or the second material clamp 812. A rotational connection method may be that a part or all of the implanting assembly 820 is connected to a housing of the storage unit 810 by rotating along its own rotation axis. Such an arrangement not only facilitates assemble the implanting assembly 820 and the storage unit 810 but also has a simple structure.

In some embodiments, the implanting assembly 820 may also slide relative to the storage unit 810 through the cooperation of a slide rail and a slide block, so that the implanting assembly 820 and the material clamps of the storage unit 810 used to store different materials may move relative to each other and output different materials accordingly.

In some embodiments, the implanting assembly 820 includes an implanting trocar 821 and a push rod 822. The implanting trocar 821 is connected to the storage unit 810, one end of the push rod 822 extends into the implanting trocar 821, and different included angles may be formed between the implanting trocar 821 and the storage unit 810 as the implanting trocar 821 rotates, so to correspond to the first material clamp 811 or the second material clamp 812. An eccentric block 823 on the push rod 822 is used to push the materials (e.g., the radioactive particle 813a or the space-occupying material 813b) in different material clamps from the material-discharging opening. In some embodiments, the eccentric block 823 is provided on the push rod 822. One end of the push rod 822 extends into the implanting trocar 821, and the eccentric block 823 corresponds to the material-discharging opening of the first material clamp 811 or the material-discharging opening of the second material clamp 812 by rotating the push rod 822 along an axis of the push rod 822. The push rod 822 pushes the radioactive particle 813a or the space-occupying material 813b in different regions of the first material clamp 811 or the second material clamp 812 to output the particle chain 813 generated by the radioactive particle 813a and/or the space-occupying material 813b in an orderly manner. The push rod 822 only pushes out one radioactive particle 813a or one space-occupying material 813b at a time. With this arrangement, the implanting assembly 820 has a simple structure and can effectively output different materials in the storage unit 810. A rotation and movement of the push rod 822 along its own axis are powered by a passive system.

The particle chain generating mechanism 800 selectively generates the particle chain 813 to be implanted by the implanting assembly 820, so that the particle chain 813 to be implanted may be adjusted and input according to individual cases, so that the particle chain 813 during the operation can be precisely controlled.

Please refer to FIG.s 10 to 12. FIG. 10 is a schematic diagram illustrating an exemplary structure of a puncture needle driving device 1000 according to some embodiments of the present disclosure. FIG. 11 is a schematic diagram illustrating an exemplary structure of the puncture needle driving device 1000 shown in FIG. 10 after being installed. FIG. 12 is a schematic diagram illustrating an exemplary structure of a puncture needle assembly 1200 according to some embodiments of the present disclosure.

Some embodiments of the present disclosure further provide the puncture needle driving device 1000. The puncture needle driving device 1000 is used to drive at least one puncture needle assembly 1200 to perform a puncture action. The puncture needle driving device 1000 includes the particle chain generating mechanism 800 and a trocar driving mechanism. In some embodiments, the trocar driving mechanism includes a first driving assembly 1010 and a second driving assembly 1020. The trocar driving mechanism is used to drive the puncture needle assembly to perform the puncture action. In some embodiments, the particle chain generating mechanism 800 may be installed inside the trocar driving mechanism. In some embodiments, a part of the particle chain generating mechanism 800 may extend out of the trocar driving mechanism, As shown in FIG. 11, the extended part of the particle chain generating mechanism 800 is the second material clamp 812 of the particle chain generating mechanism 800. The particle chain generating mechanism 800 is used to provide the particle chain 813 for the at least one puncture needle assembly 1200. The trocar driving mechanism is used to output the particle chain 813 in the at least one puncture needle assembly 1200 from an output channel 1015 to a target part. In some embodiments, when the particle chain generating mechanism 800 is installed inside the trocar driving mechanism, the particle chain 813 in the at least one puncture needle assembly 1200 may be output from the output channel 1015 on the first driving assembly 1010.

It can be understood that, in some alternative embodiments, the particle chain generating mechanism 800 may also be of other structures, as long as it may realize inputting materials such as the particle chain 813 into the at least one puncture needle assembly 1200.

Correspondingly, each of the at least one puncture needle assembly 1200 may include an outer needle 1210 and an inner needle 1220. The outer needle 1210 encases the inner needle 1220 from the outside. The first driving assembly 1010 is provided with the particle chain generating mechanism 800. The particle chain generating mechanism 800 may input the generated particle chain 813 into the outer needle 1210 of the puncture needle assembly 1200.

The outer needle 1210 is connected to the first driving assembly 1010, and the inner needle 1220 is connected to the second driving assembly 1020. In some embodiments, the first driving assembly 1010 is capable of driving an operation of the puncture needle assembly 1200, and the second driving assembly 1020 independently drives the inner needle 1220 of the puncture needle assembly 1200. In some embodiments, the first driving assembly 1010 may drive the puncture needle assembly 1200 to operate, so that the outer needle 1210 and the inner needle 1220 of the puncture needle assembly 1200 enter a target point position together. The second driving assembly 1020 may drive the inner needle 1220 of the puncture needle assembly 1200 to move axially relative to the outer needle 1210, and push the particle chain 813 located in an inner cavity of the outer needle 1210 out of the outer needle 1210, so to achieve treat the target part at the target point position. With this arrangement, materials such as the particle chain 813 in the puncture needle assembly 1200 may be freely released to the target point position.

In some embodiments, the puncture needle assembly 1200 further includes a first driving unit 1230 and a second driving unit 1240. The first driving unit 1230 is connected to the first driving assembly 1010 and the outer needle 1210 respectively, and drives the outer needle 1210 and the inner needle 1220 of the puncture needle assembly 1200 to puncture to the target point position simultaneously under an action of the first driving assembly 1010. The second driving unit is connected to the second driving assembly 1020 and the inner needle 1220 respectively, and drives the inner needle 1220 to release the particle chain 813 under the action of the second driving assembly 1020.

Specifically, the puncture needle assembly 1200 further includes the first driving unit 1230 and the second driving unit 1240 connected to each other. The first driving unit 1230 includes an outer needle installation frame 1231 and an outer needle gear 1233. The outer needle gear 1233 is connected to the outer needle installation frame 1231 and the outer needle 1210, and meshes with some components of the first driving assembly 1010. The outer needle gear 1233 is used to connect the first driving assembly 1010 and the outer needle 1210, so to drive the outer needle 1210 and the inner needle 1220 disposed in the outer needle 1210. The second driving unit 1240 includes an inner needle installation frame 1243 and an inner needle gear 1242. The inner needle gear 1242 is connected to the inner needle mounting frame 1243 and is used to drive the inner needle 1220, and the inner needle gear 1242 meshes with some components of the second driving assembly 1020, so as to drive the inner needle 1220 to push out the particle chain 813 in the outer needle 1210. Specifically, the inner needle gear 1242 is connected to an end of the inner needle 1220. Specifically, the inner needle gear 1242 is connected to the end of the inner needle 1220 in a sheathing manner. There is a screw-like structure between the inner needle gear 1242 and the inner needle 1220 so that the inner needle 1220 may move along an axial direction of the outer needle 1210. Similarly, the outer needle gear 1233 drives the outer needle 1210 using a similar principle. Such an arrangement facilitates the puncture needle driving device 1000 to accurately output the particle chain 813 in the puncture needle assembly 1200 to the target point position.

In some embodiments, the inner needle installation frame 1243 and the outer needle installation frame 1231 are stacked and snapped onto each other. The inner needle installation frame 1243 is installed on a side of the outer needle installation frame 1231 that is relatively far away from the outer needle 1210 and the inner needle 1220. When the outer needle gear 1233 drives the outer needle 1210 and the inner needle 1220 to puncture to the target point position, the inner needle gear 1242 further drives the inner needle 1220 to move along the axial direction of the outer needle 1210, and pushes the particle chain 813 in the outer needle 1210 out of the outer needle 1210 and reach the target point position. During a puncture process, the outer needle gear 1233 driven by the puncture needle driving device 1000 may drive the outer needle to rotate and insert a needle, thereby effectively reducing a resistance during a needle inserting process and the difficulty of inserting the needle. During a particle implantation process, the inner needle gear 1242 driven by the puncture needle driving device 1000 may drive the inner needle to rotate and insert a needle, thereby effectively reducing a resistance during the implantation process and the difficulty of implanting particles.

In some embodiments, the inner needle gear 1242 is installed on a side of the inner needle installation frame 1243 that is relatively away from the outer needle installation frame 1231. The puncture needle assembly 1200 further includes a needle box. The outer needle 1210, the inner needle 1220, the first driving unit 1230, and the second driving unit 1240 are all arranged in the needle box. This arrangement facilitates an overall replacement of the puncture needle assembly 1200 during a surgery. In order to further prevent the outer needle 1210 and the inner needle 1220 of the puncture needle assembly 1200 from deviating during puncture, a telescopic positioning column 1241 is provided at an end of the inner needle gear 1242 that is relatively away from the inner needle installation frame 1243. The telescopic positioning column 1241 cooperates with a positioning slot at a corresponding position of the needle box, so as to ensure the stability and puncture accuracy of the outer needle 1210, the inner needle 1220, the first driving unit 1230, and the second driving unit 1240 during surgical puncture.

In some embodiments, the outer needle installation frame 1231 has a first clamping slot 1232. A first housing 1011 of the trocar driving mechanism is provided with a first clamping part 1013. The first clamping part 1013 extends into the first clamping slot 1232 to position the outer needle 1210 on the first driving assembly 1010 of the trocar driving mechanism. The inner needle installation frame 1243 has a second clamping slot 1244. The second driving assembly 1020 is provided with a second clamping part 1023. The second clamping part 1023 extends into the second clamping slot 1244 to position the inner needle 1220 on the second driving assembly 1020 of the trocar driving mechanism. When the above two clamping parts are engaged with each other, the outer needle gear 1233 and the inner needle gear 1242 are meshed with the first driving assembly 1010 and the second driving assembly 1020, respectively. Such arrangement facilitates the docking of the first driving unit 1230 and/or the second driving unit 1240 to the corresponding first driving assembly 1010 and the second driving assembly 1020.

It can be understood that in some alternative embodiments, only one of the first clamping slot 1232 and the second clamping slot 1244 may be provided, as long as the outer needle gear 1233 and the inner needle gear 1242 may mesh with the corresponding first driving assembly 1010 and the second driving assembly 1020, respectively.

In some embodiments, the outer needle installation frame 1231 has an implantation port 1234. The implantation port 1234 is connected to the outer needle 1210. When the first driving assembly 1010 is aligned with the first driving unit 1230, the outer needle 1210 connects with the particle chain generating mechanism 800 through the implantation port 1234 and the output channel 1015 on the first driving assembly 1010. With this arrangement, the particle chain 813 or corresponding materials of the particle chain generating mechanism 800 may be smoothly input into the outer needle 1210.

In some embodiments, the first driving assembly 1010 includes the first housing 1011, a first gear 1014, and a first motor (not shown in the figure). The first gear 1014 may be driven to rotate by the first motor. The first gear 1014 is installed in the first housing 1011 and meshes with the outer needle gear 1233 to drive the outer needle 1210 of the puncture needle assembly 1200. The second driving assembly 1020 includes a second housing 1021, a second gear 1024, and a second motor (not shown in the figure). The second gear 1024 may be driven to rotate by the second motor. The second gear 1024 is installed in the second housing 1021 and meshes with the inner needle gear 1242 to drive the inner needle 1220 of the puncture needle assembly 1200. Operations of the first motor and the second motor are respectively controlled by the second processor in the aforementioned passive system.

It can be understood that the first driving assembly 1010 and/or the second driving assembly 1020 may be configured in other structures, or only one of the two assemblies may be configured.

In some embodiments, the particle chain generating mechanism 800 is disposed in the first housing 1011. The first housing 1011 is provided with the output channel 1015 and is connected to the outer needle 1210. The particle chain generating mechanism 800 communicates with the outer needle 1210 through the output channel 1015 and the implantation port 1234. When the first driving assembly 1010 and the first driving unit 1230 are docked with each other, the output channel 1015 is connected to the implantation port 1234 of the first housing 1011, so that the particle chain generating mechanism 800 may transmit the particle chain 813 to the outer needle 1210 through the output channel 1015 and the implantation port 1234.

In some embodiments, the trocar driving mechanism is also provided with a first telescopic module 1012 and a second telescopic module 1022. The first telescopic module 1012 is provided with the first driving assembly 1010, and the second telescopic module 1022 is provided with the second driving assembly 1020. The first telescopic module and the second telescopic module cooperate to align and connect the first driving assembly 1010 and the second driving assembly 1020 to the outer needle gear 1233 and the inner needle gear 1242 of the puncture needle assembly 1200. The extension and retraction of the first telescopic module 1012 and the extension and retraction of the second telescopic module 1022 are respectively controlled by the second processor in the passive system. Taking the first telescopic module 1012 as an example, when the second processor in the passive system controls the first telescopic module 1012 to be retracted into the first housing 1011, the first gear 1014 of the first driving assembly 1010 is separated from the outer needle gear 1233, so the first driving assembly 1010 is separated from the first driving unit 1230; at this time, the puncture needle assembly 1200 is completely replaced with a new puncture needle assembly 1200. After the puncture needle assembly 1200 is replaced with a new puncture needle assembly 1200, the first telescopic module extends out of the first housing 1011, and the first gear 1014 and the outer needle gear 1233 are meshed with each other. At this time, the first driving assembly 1010 and the first driving unit 1230 is in a docking state. A working principle of the second telescopic module 1022 is similar to that of the first telescopic module 1012, which is not repeated herein. This arrangement facilitates the separation and docking between the trocar driving mechanism and the puncture needle assembly 1200, thereby making the entire puncture needle assembly 1200 easy to replace.

Please refer to FIG.s 13A to 16. FIG.s 13A and 13B are schematic diagrams illustrating exemplary views of a puncture device terminal according to some embodiments of the present disclosure. FIG. 14 is a schematic diagram illustrating another exemplary structure of a puncture device terminal according to other embodiments of the present disclosure. FIG. 15 is a schematic diagram illustrating an exemplary structure of a switching frame 1310 in a puncture device terminal according to other embodiments of the present disclosure. FIG. 16 is a schematic diagram illustrating an exemplary "JL" shaped cross-section of an installation slot 1311 of the switching frame 1310 in the puncture device terminal shown in FIG. 13A.

As shown in FIG.s 13A and 13B, some embodiments of the present disclosure also provide a puncture device terminal. The puncture device terminal includes the puncture needle driving device 1000 and at least one puncture needle assembly 1200.

More descriptions regarding a specific structure of the puncture needle driving device 1000 may be found in in FIGs. 10 and 11. The puncture needle driving device 1000 is mounted on a screw structure 1330. As shown in FIG.13B, both ends of the screw structure 1330 are installed on an installation frame 41221 of the slave hand 4122. In some embodiments, the puncture needle driving device 1000 is installed on the two screw structures 1330 through an internal thread structure. In some embodiments, the two screw structures 1330 may be driven to rotate by a motor, but the puncture needle driving device 1000 does not rotate, and the puncture needle driving device 1000 moves in a longitudinal direction of the screw structure 1330.

More descriptions regarding a structure of the puncture needle assembly 1200 may be found in in FIG. 12. The puncture needle driving device 1000 is aligned and capable of driving the at least one puncture needle assembly 1200 for puncture.

In some embodiments, the puncture device terminal further includes a switching frame 1310 and a switching frame driving mechanism 1320. In some embodiments, the switching frame 1310 is installed on the installation frame 42111 of the slave hand 4122. At least two puncture needle assemblies 1200 are installed on the switching frame 1310. Moreover, as shown in FIG. 13A, the switching frame 1310 is provided with a puncture needle interface 1312 docked with the puncture needle driving device 1000. The switching frame driving mechanism 1320 switches each puncture needle assembly 1200 to the puncture needle interface 1312. The first telescopic module 1012 and the second telescopic module 1022 cooperate to connect with the puncture needle assembly 1200 at the puncture needle interface 1312. During a puncture process, the puncture needle driving device 1000 may move along an axial direction of the screw structure 1330 while driving the puncture needle assembly 1200 docked with the first telescopic module 1012 and/or the second telescopic module 1022 move axially along the screw structure 1330.

In some embodiments, the switching frame driving mechanism 1320 includes a transmission assembly. The transmission assembly is used to drive the at least one puncture needle assembly 1200 to move to the puncture needle interface 1312. The transmission assembly may be selected from at least one of a synchronous belt transmission, a gear transmission, or a chain transmission according to actual needs. The synchronous belt transmission, the gear transmission, or the chain transmission may adopt existing transmission structures. In some embodiments, the at least one puncture needle assembly 1200 moves to the puncture needle interface through translation or rotation driven by the transmission assembly.

In some embodiments, an installation slot 1311 is displaced on the switching frame. The installation slot 1311 is used for installing the at least one puncture needle assembly 1200. In some embodiments, a cross-section of the installation slot 1311 may have an annular shape (corresponding to the switching frame 1421 in FIG. 14), a spiral shape (corresponding to the switching frame 1310 in the embodiment shown in FIG. 15), or a shape of character " 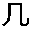 " (corresponding to the switching frame 1310 in FIG. 13A). A plurality of puncture needle assemblies 1200 are evenly arranged in the installation slot 1311. A shape of the installation slot 1311 may be selected according to actual requirements of an actual operation.

As shown in FIG. 14, in some embodiments, the cross-section of the installation slot 1311 is annular, and the switching frame 1310 has an installation surface with an annular cross-section. The installation slot 1311 is provided on the annular installation surface. The at least one puncture needle assembly 1200 disposed in the installation slot 1311 may rotate around a central axis of the annular installation surface under the driving of the switching frame driving mechanism 1320, thereby being able to continuously perform multi-needle punctures. The switching frame driving mechanism 1320 is installed on the installation frame 41221 of the slave hand 4122. The at least one puncture needle assembly 1200 may move along a longitudinal direction of the screw structure 1330 under the driver of the puncture needle driving device 1430 on the screw structure 1330. The screw structure 1330 may be installed on the installation frame 41221 of the slave hand 4122.

In some embodiments, a path of the switching frame driving mechanism 1320 switching each puncture needle assembly 1200 may include a spiral line. As shown in FIG. 15, in some embodiments, the switching frame 1310 has an installation surface 1510 with a spiral cross-section. The installation slot 1311 is provided on the installation surface 1510. In this way, the at least one puncture needle assembly 1200 disposed in the installation slot 1311 may move continuously according to a trajectory of the installation surface 1510, so as to continuously perform the multi-needle punctures.

In some embodiments, as shown in FIG. 15, the installation surface 1510 includes a first installation surface 1510a and a second installation surface 1510b. The first installation surface 1510a and the second installation surface 1510b are respectively located on an inner side and an outer side of the switching frame 1310. The inner inside of the switching frame 1310 refers to a side facing a center of the spiral line, and the outer inside of the switching frame 1310 refers to a side away from the center of the spiral line. With this arrangement, enough puncture needle assemblies 1200 may be accommodated. In some embodiments, the at least one puncture needle assembly 1200 may move along the installation surface 1510 under the driving of the switching frame driving mechanism 1320, and may move from the first installation surface 1510a to the second installation surface 1510b, or from the second installation surface 1510b to the first installation surface 1510a. In some embodiments, the installation slot1311 may be displaced on the first installation surface 1510a and the second installation surface 1510b through a crawler structure, and the installation slots 1311 on the first installation surface 1510a and the second installation surface 1510b may be connected end to end, and move in sequence around the installation surface 1510.

In some embodiments, an opening position 1520 of the installation surface 1510 may correspond to a position of the puncture needle interface 1312. The opening position 1520 may include, without limitation, the position shown in FIG. 15. When the opening position 1520 of the installation surface 1510 corresponds to the position of the puncture needle interface 1312, the switching frame driving mechanism 1320 drives the puncture needle assembly 1200 to be used to move from the installation slot 1311 to the opening position 1520 of the spiral line. The switching frame driving mechanism 1320 rotates the used puncture needle assembly 1200 from the opening position 1520 of the installation surface 1510 into the installation slot 1311.

In some embodiments, a central position 1530 of the spiral line corresponds to the position of the puncture needle interface 1312. When the center position 1530 of the spiral line corresponds to the position of the puncture needle interface 1312, the switching frame driving mechanism 1320 drives the puncture needle assembly 1200 to be used to move to the center position 1530 of the spiral line. The switching frame driving mechanism 1320 rotates the used puncture needle assembly 1200 away from the center position 1530 of the spiral line.

In some embodiments, an exemplary operation of the puncture device terminal is as follows. When a second processor of a passive system controls the slave hand 4122 to move the puncture device terminal to a designated target position, the puncture needle assembly 1200 is translated to a matching position of the puncture needle driving device 1000 with the assistance of a transmission assembly of the switching frame driving mechanism 1320, and the first telescopic module 1012 and the second telescopic module 1022 in the puncture needle driving device 1000 are docked with the first clamping slot 1232 and the second clamping slot 1244, respectively. The entire puncture needle assembly 1200 is protected by a needle box and driven by the puncture needle driving device 1000 to puncture and establish an implantation channel according to a planned path.

In some embodiments, after the implantation channel is established, the inner needle 1220 withdraws from the outer needle 1210 under an action of the second driving assembly 1020. Then, the particle chain 813 is generated using the materials in the particle chain generating mechanism 800 according to a pre-planning in an orderly manner under an action of the push rod 822, and the particle chain 813 is implanted into the outer needle 1210 through the established implantation channel. The inner needle 1220 then pushes the particle chain 813 in the outer needle 1210 out of the outer needle 1210 under an action of the second driving assembly 1020, and transmits the particle chain 813 to a target point position.

In some embodiments, after the implantation of the particle chain 813 is completed, the outer needle 1210 is withdrawn with the assistance of the corresponding first driving assembly 1010, to complete particle implantation in a needle channel. Then the first telescopic module 1012 and the second telescopic module 1022 in the puncture needle driving device 1000 are separated from the puncture needle assembly 1200, and the puncture needle assembly 1200 is recovered and released. The next puncture needle assembly 1200 arrives at a corresponding position of the puncture needle driving device 1000 with the assistance of the transmission assembly, the above steps are repeated until particle implantations of part or all of the puncture needle assemblies 1200 in the switching frame 1310 are completed.

The puncture device terminal provided in some embodiments of the present disclosure is provided with the switching frame 1310 that can switch the puncture needle assembly 1200 and the corresponding puncture drive device 1000, so that the puncture device terminal may perform multiple needle insertions on the established implantation channel at the same position, thereby enhancing the therapeutic effect and avoiding problems caused by a position deviation of the target point position of the particle chain 813 of the puncture needle assembly 1200 due to multiple needle insertions.

A working process of the puncture device terminal is basically the same compared to the working process of the puncture device terminal mentioned above, please refer to the above-mentioned descriptions.

The present disclosure also provides a surgical robot (not shown in the figures), including the particle chain generating mechanism 800 described in any of the above embodiments or the puncture device terminal described in any of the above embodiments.

In existing robotic arms, a plurality of robotic arms holds a plurality of puncture needles to complete related procedures of multi-needle punctures. The target position of the puncture needle assemblies 1200 is prone to deviation due to multiple needle insertions, thus affecting the therapeutic effect of surgery. In addition, it also increases the cost of the entire robotic arm system and prolongs the time of surgery, greatly affecting the quality and effect of surgery. Therefore, it is necessary to provide the puncture device terminal for this problem. Therefore, some embodiments of the present disclosure provide a puncture device terminal of a surgical robot, which is used to perform a surgical action on a target part.

As shown in FIG. 17, the puncture device terminal of the surgical robot may include the switching frame 1310, a switching frame driving mechanism (refers to a structure jointly formed by 1710, 1720, and 1730 in FIG. 17), and the puncture needle driving assembly 1000. At least two installation slots 1311 are disposed in the switching frame 1310. At least two installation slots 1311 are driven by the switching frame driving mechanism. At least two installation slots 1311 are used to place a plurality of corresponding puncture needle assemblies 1200. The switching frame driving mechanism is connected to the switching frame 1310, and may drive the switching frame 1310 to rotate so that the at least two puncture needle assemblies 1200 are switched. The puncture needle driving assembly 1000 is arranged on the switching frame 1310 and is used to drive the at least two puncture needle assemblies 1200 to perform a puncture action. The at least two puncture needle assemblies 1200 are installed in the installation slot 1311. Under an action of the switching frame driving mechanism, the at least two puncture needle assemblies 1200 may be switched to an electrical interface 1740 corresponding to the robotic arm one by one. The puncture device terminal may also include the electrical interface 1740. One end of the electrical interface 1740 is electrically connected to the robotic arm, and the other end is electrically connected to the puncture needle driving assembly 1000, serving as a transmission channel for electrical and control signal between the robotic arm of the surgical robot and the puncture needle driving assembly 1000. In some embodiments, an electrical connection between the electrical interface 1740 and the puncture needle driving assembly 1000 is a detachable plug-in manner, that is, the electrical interface 1740 may be connected to different puncture needle driving assemblies 1000 that are transmitted to its corresponding position. When a puncture needle assembly 1200 connected to the puncture needle driving assembly 1000 completes puncture and particle implantation operations, the electrical interface 1740 disconnects the electrical connection with the puncture needle driving assembly 1000 and waits for an electrical connection with the next puncture needle driving assembly 1000.

In some embodiments, each puncture needle assembly 1200 is connected to the puncture needle driving assembly 1000.

In some embodiments, the at least two installation slots 1311 may be arranged in an annular shape, a spiral shape, or a shape of character " 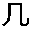 ".

In some embodiments, the switching frame 1310 may be a hollow cylindrical structure. At least two installation slots 1311 may be provided around an inner wall of the switching frame 1310. The at least two installation slots 1311 may form an end-to-end connected ring and accommodate a plurality of corresponding puncture needle assemblies 1200. Each puncture needle assembly 1200 may be switched one by one to the electrical interface 1740 corresponding to the robotic arm along a circular trajectory of the installation slot 1311 under the action of the switching frame driving mechanism.

In other embodiments, the at least two installation slots 1311 may be arranged in a spiral shape in a radially outward direction along an axis of the switching frame 1310, and each installation slot 1311 accommodates a corresponding puncture needle assembly 1200. Each puncture needle assembly 1200 may be switched one by one to the electrical interface 1740 corresponding to the robotic arm along a spiral trajectory of the installation slot 1311 under the action of the switching frame driving mechanism.

In some embodiments, the switching frame 1310 may be configured as a hollow structure with a shape of character "JL". The at least two installation slots 1311 are provided along the inner wall of the switching frame 1310. The at least two installation slots 1311 form a trajectory with a shape of character "JL" corresponding to the switching frame 1310 and accommodate a plurality of corresponding puncture needle assemblies 1200. Under the action of the switching frame driving mechanism, each puncture needle assembly 1200 may be switched one by one to the electrical interface 1740 corresponding to the robotic arm along the trajectory with a shape of character "JL" of the installation slot 1311.

In some embodiments, the switching frame 1310 may be configured as a hollow rectangular structure, and the at least two installation slots 1311 may be provided along the inner wall of the switching frame 1310. The at least two installation slots 1311 form a structure with a shape of character " 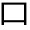 " corresponding to the switching frame 1310 and accommodate a plurality of corresponding puncture needle assemblies 1200. Each puncture needle assembly 1200 may be switched one by one to the electrical interface 1740 corresponding to the robotic arm along a trajectory with a shape of character " 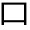 " of the installation slot 1311 under the action of the switching frame driving mechanism.

More descriptions regarding a structure of the switching frame driving mechanism may be found in in FIG. 14 and related descriptions. In some embodiments, the switching frame driving mechanism may include a rotating shaft 1710, a transmission unit 1720, and a driving component 1730. The rotating shaft 1710 may be fixedly installed on the switching frame 1310 for driving the at the least two puncture needle assemblies 1200. The transmission unit is connected to the rotating shaft 1710 and the driving component 1730, and is used to transmit a power of the driving component 1730.

Specifically, the rotating shaft 1710 may rotate under an action of the driving component 1730, and the corresponding puncture needle assembly 1200 on the switching frame 1310 is driven by the transmission unit 1720 to a hub corresponding to the robotic arm.

In some embodiments, the transmission unit 1720 may include at least one of a synchronous belt transmission, a chain transmission, or a gear transmission. The transmission unit only needs to realize power transmission between the driving component 1730 and the rotating shaft 1710, which is not limited to transmission methods in some embodiments. Multiple transmission modes expand the application scenarios, allowing the transmission unit 1720 to adapt to the requirements of the working environment and be set to at least one of the synchronous belt transmission, the chain transmission, or the gear transmission.

Each puncture needle assembly 1200 may include the outer needle 1210 and the inner needle 1220. The outer needle is accommodated in the corresponding installation slot 1311, and the inner needle 1220 is inserted into the outer needle 1210 and may move along an axial direction of the outer needle 1210 at the target position.

With this arrangement, after the outer needle 1210 completes a puncture action, the inner needle 1220 may independently perform a second puncture action inside the outer needle 1210, and push out a medical agent to be injected inside the outer needle 1210. This can avoid that a user of a puncture needle contact with the radioactive agent, thereby avoiding damage the user when the medical agent to be injected is a radioactive agent.

In some embodiments, the at the least two puncture needle assemblies 1200 may connect to the puncture needle driving assembly 1000. The puncture needle driving assembly 1000 may drive the at the least two puncture needle assemblies 1200 to move, and may independently drive the inner needle 1220 to move.

In some embodiments, the puncture needle driving assembly 1000 may adopt an existing driving structure, such as motor driving.

Specifically, the puncture needle driving assembly 1000 may include the first driving unit 1230 and the second driving unit 1240 which are relatively independently arranged. The first driving unit 1230 drives the at the least two puncture needle assemblies 1200, and the second driving unit 1240 drives the inner needle 1220. In this way, the second driving unit 1240 may independently drive the inner needle 1220 without affecting a state of the outer needle 1210, so that the inner needle 1220 may move along the axial direction of the outer needle 1210 inside the outer needle 1210, and push out the medical agent to be injected.

In some embodiments, the first driving unit 1230 and/or the second driving unit 1240 are screw drive structures. With this arrangement, the transmission efficiency of the screw drive structure is higher, and the deformation during transmission is reduced, thereby ensuring the structural stability of the first driving unit 1230 and/or the second driving unit 1240.

In some embodiments, the first driving unit 1230 and the second driving unit 1240 are electrically connected to the robotic arm respectively, so that the robotic arm may directly control the first driving unit 1230 and the second driving unit 1240.

In some embodiments, the puncture device terminal may also include the electrical interface 1740. One end of the electrical interface 1740 is electrically connected to the first driving unit 1230 and the second driving unit 1240 of the puncturing needle driving assembly 1000, respectively, and the other end is electrically connected to the robotic arm.

During a working process of the puncture device terminal of the surgical robot in some embodiments, the robotic arm first moves the switching frame 1310 to a designated position, and then drives the plurality of puncture needle assemblies 1200 to switch through the switching frame driving mechanism 1320. After a puncture needle assembly 1200 is selected, the first driving unit 1230 may release the outer needle 1210. After the outer needle 1210 completes the puncture action, the second driving unit 1240 drives the inner needle 1220 to complete the puncture action inside the outer needle 1210 and push out the medical agent to be injected. Then the first step is completed. After this, the switching frame driving mechanism 1320 may switch the puncture needle assembly 1200. The switching frame driving mechanism 1320 may switch a selected puncture needle assembly 1200 for the second puncture to the designated position, and then driving units may complete the second puncture. This action is similar to the first puncture action and will not be described in detail herein.

Compared with the prior art, some embodiments of the present disclosure provide a movable switching frame 1310 at the puncture device terminal, so that the plurality of puncture needle assemblies 1200 installed in the switching frame 1310 can be quickly switched to the target position, so that the puncture device terminal on the established puncture channel at the same position can perform a plurality of needle insertions, thereby enhancing the therapeutic effect and saving operation time.

The technical features of the above-described embodiments can be combined in any way. To simplify the description, not all possible combinations of the technical features in the above-described embodiments are described. However, as long as there is no contradiction in the combination of these technical features, should be considered as within the scope of the present disclosure.

The above-described embodiments only express several embodiments of the present disclosure, and their descriptions are relatively specific and detailed, but they should not be construed as limiting the scope of the present disclosure. It should be noted that, for those of ordinary skill in the art, several modifications and improvements can be made without departing from the concept of the present disclosure, and these all fall within the protection scope of the present disclosure. Therefore, the scope of protection of the patent in this specification should be based on the appended claims.

## Claims

1. An automated particle implantation system (41), comprising an active system (411) and a passive system (412), the active system (411) is being configured to:
obtain a scanning image of a scanned object;
obtain a radioactive particle implantation treatment planning result by planning a radioactive particle implantation treatment according to the scanning image; and
control the passive system (412) to execute a radioactive particle implantation operation according to the radioactive particle implantation treatment planning result.

2. The system of claim 1, wherein to obtain the scanning image of the scanned object, the active system (411) is further configured to:
control a scanning device (42) to scan the scanned object.

3. The system of claim 1, wherein to obtain the radioactive particle implantation treatment planning result by planning the radioactive particle implantation treatment according to the scanning image, the active system (411) is further configured to:
determine particle implantation path information based on the scanning image, a target dose, and/or a motion constraint condition of a robotic arm.

4. The system of claim 3, wherein to determine the particle implantation path information based on the scanning image, the target dose, and/or the motion constraint condition of the robotic arm, the active system (411) is further configured to:
determine a region of interest in the scanning image; and
determine the particle implantation path information according to the region of interest, the target dose, and/or the motion constraint condition of the robotic arm.

5. The system of claim 4, wherein to determine the particle implantation path information according to the region of interest, the target dose, and/or the motion constraint condition of the robotic arm, the active system (411) is further configured to:
obtain initial particle implantation path information according to a size, a shape and/or a position of the region of interest, and/or the target dose; and
determine target particle implantation path information corresponding to the scanned object by verifying the initial particle implantation path information according to the motion constraint condition of the robotic arm.

6. The system of claim 5, wherein, to determine the target particle implantation path information corresponding to the scanned object by verifying the initial particle implantation path information according to the motion constraint condition of the robotic arm, the active system (411) is further configured to:
in response to a determination that the verification passes, determine the initial particle implantation path information as the target particle implantation path information corresponding to the scanned object; and
in response to a determination that the verification fails, correct the initial particle implantation path information according to the region of interest, the target dose, and/or the motion constraint condition of the robotic arm until the verification is passed, and obtain the target particle implantation path information.

7. The system of claim 3, wherein the particle implantation path information includes a count of puncture needle assemblies, a needle insertion position of each of the puncture needle assemblies, a target point position of the each puncture needle assembly, a count of particles at the target point position of the each puncture needle assembly, and/or a moving path of the robotic arm.

8. The system of claim 7, wherein the active system (411) is further configured to:
verify one or more pieces of information of the particle implantation path information.

9. The system of claim 1, wherein the passive system (412) is configured to execute the radioactive particle implantation operation according to the radioactive particle implantation treatment planning result, the radioactive particle implantation operation comprising:
moving a robotic arm to a needle insertion position;
inserting a puncture needle assembly into a target point position;
implanting one or more particles;
withdrawing the puncture needle assembly; and
moving the robotic arm to a next needle insertion position and implanting one or more particles using a next puncture needle assembly, until all puncture needle assemblies in the radioactive particle implantation treatment planning result have been used to finish particle implantation.

10. The system of claim 1, wherein the active system (411) is further configured to:
after all puncture needle assemblies in the radioactive particle implantation treatment planning result are used to finish particle implantation, control a scanning device (42) to scan the scanned object to test an implantation effect.

11. The system of claim 1, wherein
the passive system (412) includes a puncture device;
the puncture device includes a puncture device terminal and at least one puncture needle assembly mounted on the puncture device terminal;
a size and a shape of the puncture device terminal are related to a count of the at least one puncture needle assembly; and
each of the at least one puncture needle assembly is configured to establish a particle implantation channel.

12. The system of claim 11, wherein
the active system (411) includes a radiation planning module configured to determine a count of particles according to a target dose; and
the passive system (412) includes a particle chain generating mechanism configured to form a particle chain in the particle implantation channel according to the count of particles and a space-occupying material in the radioactive particle implantation operation.

13. The system of claim 11, wherein to control the passive system (412) to execute the radioactive particle implantation operation according to the radioactive particle implantation treatment planning result, the active system (411) is further configured to:
control the passive system (412) to establish a first particle implantation channel through an outer needle of the puncture device, and inject a first particle chain into the first particle implantation channel; and
after the first particle implantation channel is established, control the passive system (412) to establish a second particle implantation channel through an inner needle of the puncture device, and inject a second particle chain into the second particle implantation channel.

14. The system of claim 1, wherein to obtain the radioactive particle implantation treatment planning result by planning the radioactive implantation treatment according to the scanning image, the active system (411) is further configured to:
obtain a body surface position of the scanned object through a scanning device (42); and
in response to a determination that a change amount of the body surface position within a preset time is greater than or equal to a preset change threshold, modify the radioactive particle implantation treatment planning result according to a current scanning image.

15. The system of claim 1, wherein the active system (411) includes a display device configured to display at least one of the scanning image, the radioactive particle implantation treatment planning result, a remaining amount of particles to be implanted, or a motion state of the passive system (412).

16. A particle chain generating mechanism (800), comprising a storage unit (810) and an implanting assembly (820), wherein,
the storage unit (810) is configured to store at least two materials used to form a particle chain (813); and
at least a portion of the implanting assembly (820) is inserted into the storage unit (810) and can selectively cause the at least two materials located in the storage unit (810) to form the particle chain (813) in an orderly manner.

17. The mechanism of claim 16, wherein the at least two materials include a radioactive particle (813a) and a space-occupying material (813b).

18. The mechanism of claim 17, wherein the storage unit (810) includes a first material clamp (811) and a second material clamp (812);
the first material clamp (811) is configured to store the radioactive particle (813a); and
the second material clamp (812) is configured to store the space-occupying material (813b).

19. The mechanism of claim 18, wherein the implanting assembly (820) is selectively connected to the first material clamp (811) or the second material clamp (812).

20. The mechanism of claim 19, wherein at least a portion of the implanting assembly (820) is rotatably connected to the storage unit (810), so as to be selectively connected to the first material clamp (811) or the second material clamp (812).

21. The mechanism of claim 19, wherein
the implanting assembly (820) includes an implanting trocar (821) and a push rod (822), and the implanting trocar (821) is rotatably connected to the storage unit (810); and
an end of the push rod (822) extends into the implanting trocar (821) and at least two included angles between the storage unit (810) and the implanting trocar (821) are formed as the implanting trocar (821) rotates, so as to correspond to the first material clamp (811) or the second material clamp (812).

22. A puncture device terminal, comprising a switching frame (1310) and a switching frame driving mechanism (1320), wherein the switching frame driving mechanism (1320) includes a transmission assembly configured to drive at least one puncture needle assembly (1200) to move to a puncture needle interface (1312).

23. The puncture device terminal of claim 22, wherein the at least one puncture needle assembly (1200) is driven by the transmission assembly to move to the puncture needle interface (1312) in a translational or rotational manner.

24. The puncture device terminal of claim 22, wherein the at least one puncture needle assembly (1200) includes at least two puncture needle assemblies (1200), and the switching frame (1310) is provided with an installation slot (1311) configured to install the at least two puncture needle assemblies (1200).

25. The puncture device terminal of claim 24, wherein a cross-section of the installation slot (1311) has an annular shape, a spiral shape, or a shape of character " 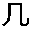 ".

26. The puncture device terminal of claim 22, further comprising a puncture needle driving device (1000), wherein the puncture needle driving device (1000) includes a particle chain generating mechanism (800) and a trocar driving mechanism, the particle chain generating mechanism (800) is configured to provide a particle chain (813) for the at least one puncture needle assembly (1200), and the trocar driving mechanism is configured to drive the at least one puncture needle assembly (1200) to perform a puncture operation.

27. The puncture device terminal of claim 26, wherein each of the at least one puncture needle assembly (1200) includes an outer needle (1210) and an inner needle (1220), and the outer needle (1210) covers the inner needle (1220).

28. The puncture device terminal of claim 27, wherein
each of the at least one puncture needle assembly (1200) further includes a first driving unit (1230) and a second driving unit (1240);
the first driving unit (1230) connects and drives the outer needle (1210) and the inner needle (1220) to perform the puncture operation; and
the second driving unit (1240) connects and drives the inner needle (1220) to release the particle chain (813) provided by the particle chain generating mechanism (800).

29. A puncture device terminal, comprising a switching frame (1310) and a switching frame driving mechanism (1320), wherein
the switching frame (1310) is provided with an installation slot (1311) and a puncture needle interface (1312) connected to the installation slot (1311),
the installation slot (1311) is configured to install at least one puncture needle assembly (1200); and
the switching frame driving mechanism (1320) is configured to switch each of the at least one puncture needle assembly (1200) to the puncture needle interface (1312).

30. The puncture device terminal of claim 29, wherein a path of the switching frame driving mechanism switching each of the at least one puncture needle assembly (1200) includes a spiral line.

31. The puncture device terminal of claim 30, wherein the switching frame (1310) has an installation surface (1510) with a spiral cross-section, and the installation slot (1311) is provided on the installation surface (1510).

32. The puncture device terminal of claim 31, wherein the installation surface (1510) includes a first installation surface (1510a) and a second installation surface (1510b), the first installation surface (1510a) and the second installation surface (1510b) are located on an inner side and an outer side of the switching frame (1310), respectively.

33. The puncture device terminal of claim 31, wherein an opening position of the installation surface (1510) corresponds to a position of the puncture needle interface (1312), or a center position of the spiral line corresponds to the position of the puncture needle interface (1312).

34. A puncture device terminal, comprising:
a switching frame (1310) configured to connect with a robotic arm;
at least two installation slots (1311) provided in the switching frame (1310), the at least two installation slots (1311) being configured to accommodate at least two puncture needle assemblies (1200);
a switching frame driving mechanism (1320) provided in the switching frame (1310), the switching frame driving mechanism (1320) being configured to drive the at least two puncture needle assemblies (1200) to switch; and
a puncture needle driving device (1000) configured to drive the at least two puncture needle assemblies (1200) to perform one or more puncture operations.

35. The puncture device terminal of claim 34, wherein the switching frame (1310) rotates under the driving of the switching frame driving mechanism (1320).

36. The puncture device terminal of claim 35, wherein
the switching frame driving mechanism (1320) includes a rotating shaft (1710), a transmission unit (1720), and a driving component (1730);
the rotating shaft (1710) is fixedly installed on the switching frame (1310); and
the transmission unit (1720) is connected to the rotating shaft (1710) and the driving component (1730).

37. The puncture device terminal of claim 36, wherein the transmission unit (1720) includes at least one of a synchronous belt transmission, a chain transmission, or a gear transmission.

38. The puncture device terminal of claim 34, wherein
each of the at least two puncture needle assemblies (1200) includes an outer needle (1210) and an inner needle (1220);
the outer needle (1210) is accommodated in the installation slot (1311); and
the inner needle (1220) is inserted into the outer needle (1210) and can move along an axial direction of the outer needle (1210).

39. The puncture device terminal of claim 38, wherein
the puncture needle driving device (1000) includes a first driving unit (1230) and a second driving unit (1240) arranged independently from each other;
the first driving unit (1230) drives the outer needle (1210) and the inner needle (1220); and
the second driving unit (1240) drives the inner needle (1220).

40. The puncture device terminal of claim 39, wherein the first driving unit (1230) and the second driving unit (1240) are screw drive structures.

41. The puncture device terminal of claim 39, wherein the first driving unit (1230) and the second driving unit (1240) are electrically connected to the robotic arm, respectively.

42. The puncture device terminal of claim 41, further comprising an electrical interface (1740), wherein
the electrical interface (1740) is provided on the switching frame (1310); one end of the electrical interface (1740) is electrically connected to the first driving unit (1230) and the second driving unit (1240) respectively; and
the other end of the electrical interface (1740) is electrically connected to the robotic arm.

43. The puncture device terminal of claim 34, wherein the puncture needle driving device (1000) is provided on the switching frame (1310).

44. The puncture device terminal of claim 34, wherein the at least two installation slots (1311) are driven by the switching frame driving mechanism (1320).

45. The puncture device terminal of claim 34, wherein the at least two installation slots (1311) are arranged in an annular shape, a spiral shape, or a shape of character " 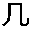 ".
